(19) Europäisches Patentamt
European Patent Office
Office européen des brevets

(11) Veröffentlichungsnummer: **0 463 492 A1**

# (12) EUROPÄISCHE PATENTANMELDUNG

(21) Anmeldenummer: **91109714.5**

(22) Anmeldetag: **13.06.91**

(51) Int. Cl.[5]: **C07D 213/68**, C07D 213/71, C07D 213/74

(30) Priorität: **26.06.90 DE 4020257**

(43) Veröffentlichungstag der Anmeldung: **02.01.92 Patentblatt 92/01**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Yanagi, Akihiko, Dr.**
**2-1200-1, Nagabuchi**
**Oume-Chi, Tokyo(JP)**
Erfinder: **Heinemann, Ulrich, Dr.**
**Feldstrasse 18**
**W-5653 Leichlingen 1(DE)**
Erfinder: **Babczinski, Peter, Dr.**
**In der Lohrenbeck 11**
**W-5600 Wuppertal 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **2,6-Diarylpyridin-Derivate.**

(57) 2,6-Diarylpyridin-Derivate der allgemeinen Formel (I),

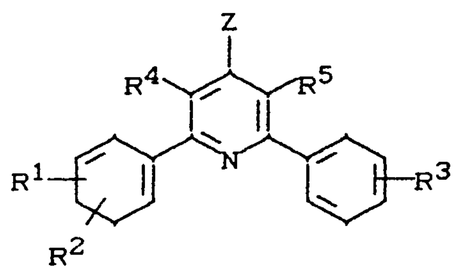

(I)

in welcher

$R^1$ und $R^2$ jeweils unabhängig voneinander für Wasserstoff, Halogen, Alkyl, Alkoxy oder Halogenalkyl stehen,

$R^3$ für Wasserstoff, Halogen, Cyano, Alkyl, Alkoxy oder Halogenalkyl steht,

$R^4$ und $R^5$ jeweils unabhängig voneinander für Wasserstoff oder Alkyl stehen und

Z für Alkyl, Alkenyl, Alkinyl, Alkoxy, Alkenyloxy, Alkinyloxy, Alkylthio, Alkenylthio, Alkinylthio, Alkoxycarbonylalkylthio, Trimethylsilylalkinyl, Halogen oder für eine der folgenden Gruppierungen

$-NH-R^6$;

EP 0 463 492 A1

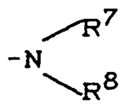 oder 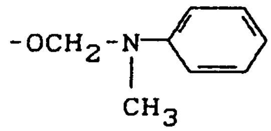

steht, mehrere Verfahren und neue Zwischenprodukte zu deren Herstellung und ihre Verwendung als Herbizide.

Die Erfindung betrifft neue 2,6-Diarylpyridin-Derivate, neue Zwischenprodukte sowie mehrere Verfahren zu ihrer Herstellung und ihre Verwendung als Herbizide.

Es ist bereits bekannt, daß bestimmte, in 4-Stellung substituierte 2,6-Diphenylpyridin-Derivate, herbizide Eigenschaften besitzen (vergl. EP-A 263 958).

Die Wirksamkeit dieser oben genannten Verbindungen ist jedoch nicht in allen Anwendungsgebieten völlig zufriedenstellend.

Weiterhin werden in 4-Stellung substituierte 2,6-Diphenylpyridin-Derivate als Zwischenprodukte für die Herstellung von Antimalariamitteln (vergl. US-P 3 600 396) sowie für die Synthese von Pyrylium-Salzen (vergl. US-P 4 451 659) beschrieben.

Darüberhinaus wird die Synthese von in 4-Stellung substituierten 2,6-Diphenylpyridin-Derivaten in der Literatur mehrfach beschrieben (vergl. z.B. J. Org. Chem. 47 (16), 3027-3038, 1982 und die beim Disclaimer angegebene Literatur).

Ihre Verwendung in der Landwirtschaft wird allerdings nicht erwähnt.

Es wurden neue 2,6-Diarylpyridin-Derivate der allgemeinen Formel (I) gefunden

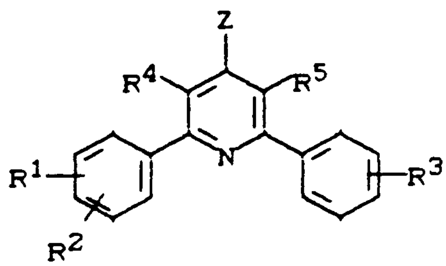

(I)

in welcher

$R^1$ und $R^2$ jeweils unabhängig voneinander für Wasserstoff, Halogen, Alkyl, Alkoxy oder Halogenalkyl stehen,

$R^3$ für Wasserstoff, Halogen, Cyano, Alkyl, Alkoxy oder Halogenalkyl steht,

$R^4$ und $R^5$ jeweils unabhängig voneinander für Wasserstoff oder Alkyl stehen und

Z für Alkyl, Alkenyl, Alkinyl, Alkoxy, Alkenyloxy, Alkinyloxy, Alkylthio, Alkenylthio, Alkinylthio, Alkoxycarbonylalkylthio, Trimethylsilylalkinyl, Halogen oder für eine der folgenden Gruppierungen

$-NH-R^6$;

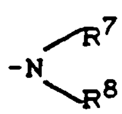

oder

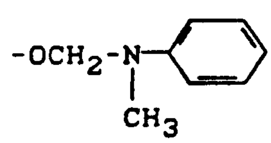

steht, wobei

$R^6$ und $R^7$ für Alkyl, Alkenyl oder Alkinyl stehen,

$R^8$ für Alkyl steht oder

$R^7$ und $R^8$ gemeinsam mit dem Stickstoffatom, an welches sie gebunden sind, für eine gegebenenfalls durch Sauerstoff, Schwefel oder für durch Alkyl substituierten Stickstoff unterbrochene gesättigte Alkylkette stehen,

wobei die Verbindungen ausgenommen sind, in denen

$R^4$ und $R^5$ gleichzeitig für Wasserstoff stehen,

Z für Alkoxy oder Alkylthio steht und

a) $R^1$ und $R^2$ gleichzeitig für Wasserstoff stehen oder

b) einer der Reste $R^1$ oder $R^2$ für Wasserstoff steht und der andere Rest $R^2$ bzw. $R^1$ in der ortho- oder meta-Position des Phenyl für Halogen, Methyl oder Trifluormethyl steht,

[vergl. EP-A 263 958, J. Org. Chem. 47 (16) 3027-3038 (1982), J. Am. Chem. Soc. 90 (6), 1569-1582 (1968), J. Am. Chem. Soc. 103 (12), 3585-3586 (1981)]

und wobei die Verbindungen

2,6-Bis(4-bromphenyl)-4-methylthio-pyridin,

2,6-Bis(4-methoxyphenyl)-4-methylthio-pyridin,
4-n-Butyl-2,6-diphenyl-pyridin,
4-(1-Methylethyl)-2,6-diphenyl-pyridin,
2,6-Bis(4-methoxyphenyl)-4-methyl-pyridin,
4-Methyl-2,6-diphenyl-pyridin,
2,6-Bis(p-chlorphenyl)-4-picolin und
4-Ethyl-2,6-diphenyl-pyridin
ausgenommen sind
(vergl. J. Am. Chem. Soc. 103 (12), 3585-3586 (1981), J. Org. Chem. 50 (7) 1125-1126 (1985), Chem. Abst. 102 (15): 131 894s, US-P 3 600 396 und Liebigs Ann. Chem. 625 (1959) 74-88).

Weiterhin wurde gefunden, daß man die neuen 2,6-Diarylpyridin-Derivate der Formel (I)

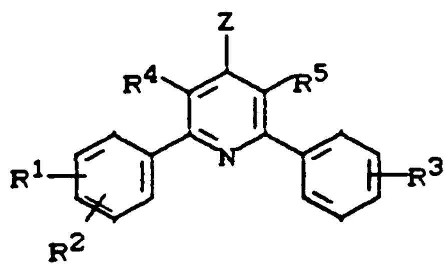

(I)

in welcher

$R^1$ und $R^2$ jeweils unabhängig voneinander für Wasserstoff, Halogen, Alkyl, Alkoxy oder Halogenalkyl stehen,
$R^3$ für Wasserstoff, Halogen, Cyano, Alkyl, Alkoxy oder Halogenalkyl steht,
$R^4$ und $R^5$ jeweils unabhängig voneinander für Wasserstoff oder Alkyl stehen und
Z für Alkyl, Alkenyl, Alkinyl, Alkoxy, Alkenyloxy, Alkinyloxy, Alkylthio, Alkenylthio, Alkinylthio, Alxoxycarbonylalkylthio, Trimethylsilylalkinyl, Halogen oder für eine der folgenden Gruppierungen
$-NH-R^6$;

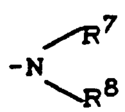

oder

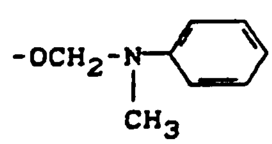

steht, wobei

$R^6$ und $R^7$ für Alkyl, Alkenyl oder Alkinyl stehen,
$R^8$ für Alkyl steht oder
$R^7$ und $R^8$ gemeinsam mit dem Stickstoffatom, an welches sie gebunden sind, für eine gegebenenfalls durch Sauerstoff, Schwefel oder für durch Alkyl substituierten Stickstoff unterbrochene gesättigte Alkylkette stehen,

ausgenommen die bereits oben genannten Verbindungen, nach einem der im folgenden beschriebenen Verfahren erhält:

a) Man erhält die erfindungsgemäßen 2,6-Diarylpyridin-Derivate der Formel (Ia)

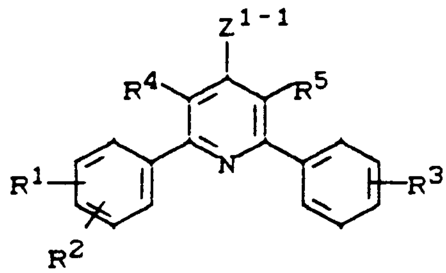

(Ia)

in welcher

$R^1$, $R^2$, $R^3$, $R^4$ und $R^5$ die oben angegebene Bedeutung haben und

$Z^{1-1}$ für Alkylthio, Alkenylthio, Alkinylthio oder Alkoxycarbonylalkylthio steht,

wenn man 1,5-Dioxo-Verbindungen der Formel (II) bzw. deren Isomere der Formel (IIa)

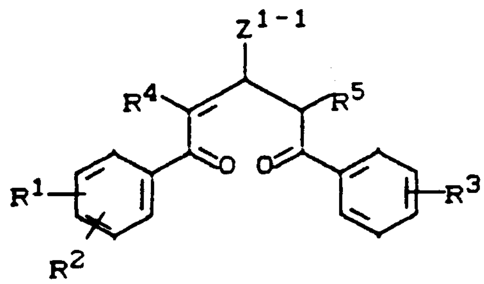

(II)

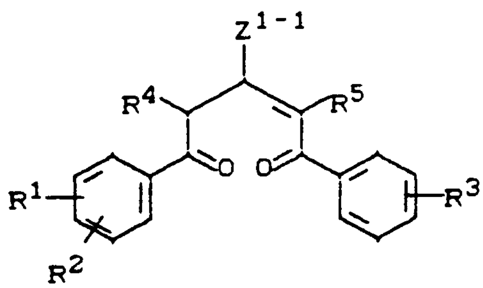

(IIa)

in welcher

$R^1$, $R^2$, $R^3$, $R^4$, $R^5$ und $Z^{1-1}$ die oben angegebene Bedeutung haben,

mit Ammoniak oder einem Ammoniumsalz gegebenenfalls in Gegenwart eines Verdünnungsmittels umsetzt;

b) Man erhält die erfindungsgemäßen 2,6-Diarylpyridin-Derivate der Formel (Ib)

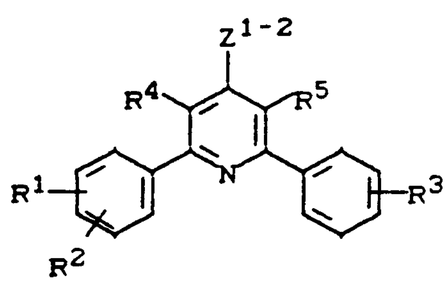

(Ib)

in welcher

$Z^{1-2}$ für Alkoxy, Alkenyloxy, Alkinyloxy oder für eine der folgenden Gruppierun-

gen
-NH-$R^6$;

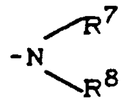 oder 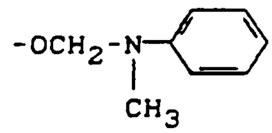

steht und

$R^1$, $R^2$, $R^3$, $R^4$ und $R^5$ die oben angegebene Bedeutung haben,

wenn man 4-Sulfonylalkyl-2,6-diarylpyridin-Derivate der Formel (III)

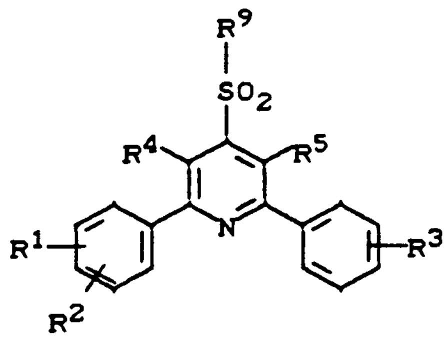 (III)

in welcher

$R^1$, $R^2$, $R^3$, $R^4$ und $R^5$ die oben angegebene Bedeutung haben und

$R^9$ für Alkyl steht, entweder

b-$\alpha$) mit Verbindungen der Formel (IV)

$Z^{1-3}$-M (IV)

in welcher

$Z^{1-3}$ für Alkoxy, Alkenyloxy, Alkinyloxy oder für die Gruppe

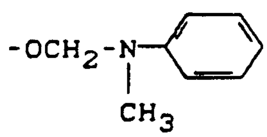

steht und

M für ein Alkalimetallkation steht,

gegebenenfalls in Gegenwart eines Verdünnungsmittels umsetzt, oder

b-$\beta$) mit Aminen der Formel (V) oder (Va)

$H_2N-R^6$ (V)

oder

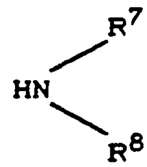 (Va)

in welcher

$R^6$, $R^7$ und $R^8$ die oben angegebene Bedeutung haben,

gegebenenfalls in Gegenwart eines Verdünnungsmittels und gegebenenfalls unter erhöhtem Druck umsetzt;

c) Man erhält die erfindungsgemäßen 2,6-Diarylpyridin-Derivate der Formel (Ic)

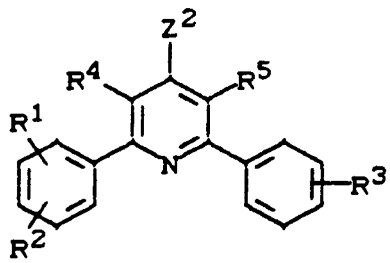 (Ic)

in welcher

$R^1$, $R^2$, $R^3$, $R^4$ und $R^5$ die oben angegebene Bedeutung haben und

$Z^2$ für Halogen steht,

wenn man 4-Hydroxy-2,6-Diarylpyridin-Derivate der Formel (VI)

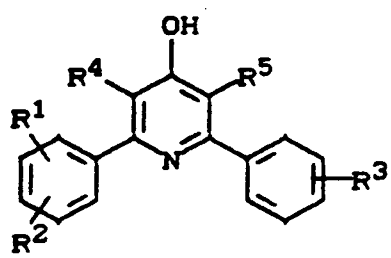 (VI)

in welcher

$R^1$, $R^2$, $R^3$, $R^4$ und $R^5$ die oben angegebene Bedeutung haben,

mit einem Halogenierungsreagenz gegebenenfalls in Gegenwart eines Verdünnungsmittels umsetzt.

d) Man erhält die erfindungsgemäßen 2,6-Diarylpyridin-Derivate der Formel (Id)

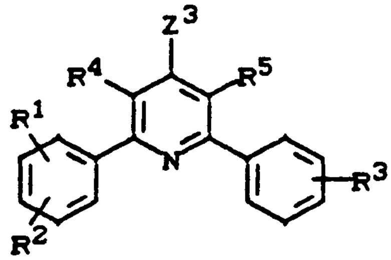 (Id)

in welcher

$R^1$, $R^2$, $R^3$, $R^4$ und $R^5$ die oben angegebene Bedeutung haben, und

$Z^3$ für Alkyl, Alkenyl, Alkinyl oder Trimethylsilylalkinyl steht,

wenn man
die erfindungsgemäßen 2,6-Diarylpyridin-Derivate der Formel (Ic)

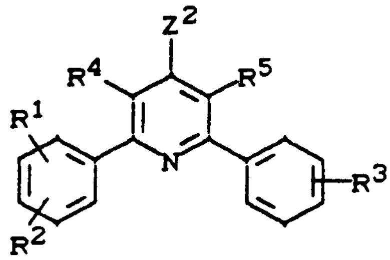

(Ic)

in welcher
$R^1$, $R^2$, $R^3$, $R^4$ und $R^5$ die oben angegebene Bedeutung haben, und
$Z^2$ für Halogen steht,
mit Diester-Derivaten der Formel (VII)

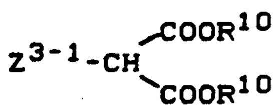

(VII)

in welcher
$Z^{3-1}$ für Wasserstoff, Alkyl, Alkenyl, Alkinyl oder Trimethylsilylalkinyl steht, und
$R^{10}$ für Methyl oder Ethyl steht, gegebenenfalls in Gegenwart eines Verdünnungsmittels und gegebenenfalls in Gegenwart einer Base umsetzt und die so erhaltenen Verbindungen gegebenenfalls in Gegenwart eines Verdünnungsmittels und in Gegenwart einer Base hydrolysiert und anschließend in Gegenwart einer Säure decarboxyliert.

Schließlich wurde gefunden, daß die neuen 2,6-Diarylpyridin-Derivate der Formel (I) eine sehr gute herbizide Wirksamkeit besitzen.

Die Reste ($R^1$, $R^2$ usw.) die in den Wirkstoffen der Formel (I) definiert sind, haben in den Zwischen- und Vorprodukten für alle Definitionsbereiche ebenfalls die bei den Verbindungen der Formel (I) angegebenen Bedeutungen. Entsprechendes gilt auch für die Reste, die in Vor- und Zwischenprodukten mehrfach genannt sind.

Die Kohlenstoffkette in Alkyl, Alkoxy, Alkylthio, Alkenyl usw. oder in Zusammensetzungen wie Alkoxycarbonylalkylthio ist jeweils geradkettig oder verzweigt.

Für den Begriff Alkyl in den Definitionen von $R^1$, $R^2$, $R^3$, $R^4$, $R^5$, $R^6$, $R^7$ und $R^8$ steht geradkettiges oder verzweigtes Alkyl mit vorzugsweise 1 bis 6, besonders bevorzugt 1 bis 4 und insbesondere 1 oder 2 Kohlenstoffatomen. Beispielhaft seien Methyl, Ethyl, n-Propyl, i-Propyl, n-Butyl, sec-Butyl, i-Butyl, t-Butyl, n-Pentyl, i-Pentyl und t-Pentyl genannt.

Für den Begriff Alkoxy in der Definition von $R^1$, $R^2$ und $R^3$ steht geradkettiges oder verzweigtes Alkoxy mit vorzugsweise 1 bis 6, besonders bevorzugt 1 bis 4 Kohlenstoffatomen je Alkylrest; beispielhaft und vorzugsweise seien genannt: Methoxy, Ethoxy, n- und i-Propoxy, n-, i-, s-und t-Butoxy, n-Hexoxy und i-Hexoxy.

Für den Begriff Alkyl in der Definition von Z steht geradkettiges oder verzweigtes Alkyl mit vorzugsweise 1 bis 8, besonders 1 bis 6 und insbesondere mit 1 bis 4 Kohlenstoffatomen. Beispielhaft seien genannt: Methyl, Ethyl, n- oder i-Propyl, n-, i-, s- oder t-Butyl.

Für den Betriff Alkoxy in der Definition von Z steht geradkettiges oder verzweigtes Alkoxy mit vorzugsweise 1 bis 6 und insbesondere mit 1 bis 4 Kohlenstoffatomen. Beispielhaft seien genannt: Methoxy, Ethoxy, n- oder i-Propoxy, n-- i-, s- oder t-Butoxy.

Für den Begriff Halogenalkyl steht in den Definitionen von $R^1$, $R^2$ und $R^3$ geradkettiges oder verzweigtes Alkyl mit vorzugsweise 1 bis 6 Kohlenstoffatomen, besonders bevorzugt mit 1 bis 4 Kohlenstoffatomen und 1 bis 13, vorzugsweise 1 bis 6, gleichen oder verschiedenen Halogenatomen, beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluormethyl, Trifluorchlorethyl, Chlorbutyl, Fluorbutyl.

Der Begriff Halogen steht in den Definitionen von $R^1$, $R^2$, $R^3$ und Z im allgemeinen für Fluor, Chlor, Brom und Iod, bevorzugt für Fluor, Chlor und Brom und besonders bevorzugt für Fluor und Chlor.

Für die Begriffe Alkenyl und Alkinyl selbst, sowie für Zusammensetzungen wie Alkenyloxy, Alkinyloxy, Alkenylthio, Alkinylthio oder Trimethylsilylalkinyl, stehen in den Definitionen von $R^6$, $R^7$ und Z im allgemeinen geradkettiges oder verzweigtes Alkenyl oder Alkinyl mit vorzugsweise 2 bis 6, besonders bevorzugt 2 bis 4 und ganz besonders bevorzugt 2 oder 3, Kohlenstoffatomen, Beispielhaft seien Vinyl, Allyl, Propenyl-(2)-, 1-Butenyl, 2-Butenyl, 3-Butenyl, Ethinyl, 1-Propinyl, 2-Propinyl, 1-Butinyl, 2-Butinyl und 3-Butinyl genannt.

Für den Fall, daß in der Definition von Z die beiden Reste $R^7$ und $R^8$ gemeinsam mit dem Stickstoffatom, an welches sie gebunden sind, einen Ring bilden, enthält dieser 5 bis 7, vorzugsweise 5 oder 6 Ringglieder. Der Ring kann ein weiteres Heteroatom, vorzugsweise Sauerstoff, Schwefel oder Stickstoff enthalten. Beispielhaft seien genannt: Pyrrolidino, Piperidino, Morpholino, N-Methylpiperazino und Thiomorpholino.

Alkylthio steht im allgemeinen in dem Rest Z für geradkettiges oder verzweigtes Alkylthio mit vorzugsweise 1 bis 6 Kohlenstoffatomen, beispielsweise sind darunter die folgenden Gruppen zu verstehen: Methylthio-, Ethylthio-, Propylthio-, Butylthio-, Pentylthio sowie ihre Isomeren, wie z.B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Alkoxycarbonylalkylthio steht im allgemeinen für geradkettiges oder verzweigtes Alkoxycarbonylalkylthio mit 1 bis 6, vorzugsweise 1 bis 4 Kohlenstoffatomen im Alkoxyrest und 1 bis 4, vorzugsweise 1 bis 2, Kohlenstoffatomen im Alkylrest; beispielhaft seien genannt; Methoxycarbonylmethylthio, Ethoxycarbonylmethylthio, n-Propoxycarbonylmethylthio, i-Propoxycarbonylmethylthio, n-, i-, s- und t-Butoxycarbonylmethylthio.

Bevorzugt sind Verbindungen der Formel (I), bei welchen

- $R^1$ für Wasserstoff, Halogen, Alkyl oder Alkoxy mit jeweils 1 bis 6 Kohlenstoffatomen oder für Halogenalkyl mit 1 bis 6 Kohlenstoffatomen und 1 bis 13 gleichen oder verschiedenen Halogenatomen steht,
- $R^2$ für Wasserstoff, Halogen, Alkyl oder Alkoxy mit jeweils 1 bis 6 Kohlenstoffatomen oder für Halogenalkyl mit 1 bis 6 Kohlenstoffatomen und 1 bis 13 gleichen oder verschiedenen Halogenatomen steht,
- $R^3$ für Wasserstoff, Halogen, Cyano, Alkyl oder Alkoxy mit jeweils 1 bis 6 Kohlenstoffatomen oder für Halogenalkyl mit 1 bis 6 Kohlenstoffatomen und 1 bis 13 gleichen oder verschiedenen Halogenatomen steht,
- $R^4$ für Wasserstoff oder Alkyl mit 1 bis 6 Kohlenstoffatomen steht,
- $R^5$ für Wasserstoff oder Alkyl mit 1 bis 6 Kohlenstoffatomen steht,
- Z für Alkyl mit 1 bis 8 Kohlenstoffatomen, Alkoxy oder Alkylthio mit jeweils 1 bis 8, insbesondere 1 bis 6 Kohlenstoffatomen, oder für Alkenyl, Alkenyloxy oder Alkenylthio mit jeweils 2 bis 6 Kohlenstoffatomen, Alkinyl, Alkinyloxy oder Alkinylthio mit jeweils 2 bis 6 Kohlenstoffatomen, Alkoxycarbonylalkylthio mit jeweils 1 bis 4 Kohlenstoffatomen im Alkoxy- oder Alkylteil, Trimethylsilylalkinyl mit 2 bis 4 Kohlenstoffatomen im Alkinylteil, Halogen oder für eine der folgenden Gruppierungen
  $-NH-R^6$;

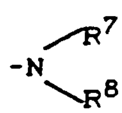

oder

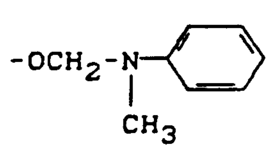

steht, wobei

- $R^6$ und $R^7$ unabhängig voneinander für Alkyl mit 1 bis 6 Kohlenstoffatomen, Alkenyl mit 2 bis 6 Kohlenstoffatomen oder Alkinyl mit 2 bis 6 Kohlenstoffatomen steht,
- $R^8$ für Alkyl mit 1 bis 6 Kohlenstoffatomen steht oder
- $R^7$ und $R^8$ gemeinsam mit dem Stickstoffatom, an welches sie gebunden sind, einen 5- oder 6-gliedrigen gesättigten Ring bilden, welcher gegebenenfalls ein Sauerstoff-, Schwefel- oder Stickstoffatom enthält,

wobei das Stickstoffatom durch $C_1$-$C_4$-Alkyl, insbesondere durch Methyl, substituiert sein kann,

ausgenommen die bereits oben genannten Verbindungen.

Besonders bevorzugt sind Verbindungen der Formel (I), bei welchen

$R^1$ für Wasserstoff, Fluor, Chlor, Brom, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy oder für Halogenalkyl mit 1 bis 4 Kohlenstoffatomen und 1 bis 9 Fluor- und/oder Chloratomen steht,

$R^2$ für Wasserstoff, Fluor, Chlor, Brom, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy oder für Halogenalkyl mit 1 bis 4 Kohlenstoffatomen und 1 bis 9 Fluor- und/oder Chloratomen steht,

$R^3$ für Wasserstoff, Fluor, Chlor, Brom, Cyano, Methyl, Ethyl, n-Propyl, i-Propyl, n-Butyl-, i-Butyl, s-Butyl, t-Butyl, Methoxy, Ethoxy, n-Propoxy, i-Propoxy, n-Butoxy, i-Butoxy, s-Butoxy, t-Butoxy oder für Halogenalkyl mit 1 bis 4 Kohlenstoffatomen und 1 bis 9 Fluor- und/oder Chloratomen steht,

$R^4$ und $R^5$ unabhängig voneinander für Wasserstoff, Methyl, Ethyl, n-Propyl, i-Propyl, n-Butyl, i-Butyl, s-Butyl oder t-Butyl stehen,

Z für Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy, Methylthio, Ethylthio, n-Propylthio, i-Propylthio, n-Butylthio, s-Butylthio, i-Butylthio, t-Butylthio, für Alkenyl, Alkenyloxy oder Alkenylthio mit jeweils 2 bis 4 Kohlenstoffatomen, Alkinyl, Alkinyloxy oder Alkinylthio mit 2 bis 4 Kohlenstoffatomen, Alxoxycarbonylalkylthio mit 1 bis 4 Kohlenstoffatomen im Alkoxy- und 1 oder 2 Kohlenstoffatomen im Alkylteil, Trimethylsilylalkinyl mit 2 bis 4 Kohlenstoffatomen im Alkinylteil, Fluor, Chlor, Brom oder für eine der folgenden Gruppierungen

$-NH-R^6$;

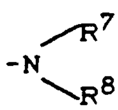 oder 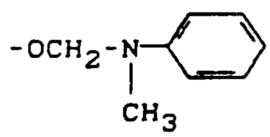

steht, wobei

$R^6$ und $R^7$ unabhängig voneinander für Methyl, Ethyl, n-Propyl, i-Propyl, n-Butyl, i-Butyl, s-Butyl, t-Butyl, Alkenyl mit 2 bis 4 Kohlenstoffatomen oder Alkinyl mit 2 bis 4 Kohlenstoffatomen steht,

$R^8$ für Methyl, Ethyl, n-Propyl, i-Propyl, n-Butyl, i-Butyl, s-Butyl oder t-Butyl steht oder

$R^7$ und $R^8$ gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält, wobei das Stickstoffatom durch Methyl, Ethyl, n- oder i-Propyl substituiert sein kann, insbesondere steht $R^7$ und $R^8$ zusammen für $-(CH_2)_4-$, $-(CH_2)_5-$, $-CH_2-CH_2-O-CH_2-CH_2-$ oder

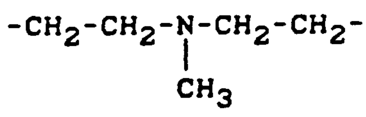

ausgenommen die bereits oben genannten Verbindungen.

Ganz besonders bevorzugt sind Verbindungen der Formel (I), bei welchen

$R^1$ für Wasserstoff, Fluor, Chlor, Methyl, Ethyl, n-Propyl, i-Propyl, t-Butyl, Methoxy, Ethoxy, Trifluormethyl, Difluormethyl oder Trichlormethyl steht,

$R^2$ für Wasserstoff, Fluor, Chlor, Methyl, Ethyl, Methoxy, Ethoxy, Trifluormethyl, Difluormethyl oder Trichlormethyl steht,

$R^3$ für Wasserstoff, Fluor, Chlor, Brom, Cyano, Methyl, Ethyl, Methoxy, Ethoxy, Trifluormethyl, Difluormethyl oder Trichlormethyl steht,

$R^4$ für Wasserstoff, Methyl oder Ethyl steht,

$R^5$ für Wasserstoff, Methyl oder Ethyl steht,

Z für Methyl, Ethyl, n-Propyl, i-Propyl, Methoxy, Ethoxy, n-Propoxy, i-Propoxy, Methylthio, Ethylthio, n-Propylthio, i-Propylthio, Allyl, Allyloxy, Allylthio, Propargyl, Propargyloxy, Propargylthio, Ethinyl, Ethinyloxy, Ethinylthio, Methoxycarbonylmethylthio, Ethoxycarbonylmethylthio, Fluor, Chlor oder für eine der folgenden Gruppierungen

$-C{\equiv}C-Si(CH_3)_3$ ; $-NH-R^6$ ;

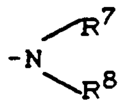 oder

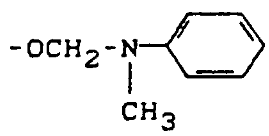

steht, wobei

$R^6$ für Methyl, Ethyl, n-Propyl oder i-Propyl steht,

$R^7$ für Methyl, Ethyl, n-Propyl oder i-Propyl steht,

$R^8$ für Methyl, Ethyl, n-Propyl oder i-Propyl steht oder

$R^7$ und $R^8$ gemeinsam mit dem Stickstoffatom, an welches sie gebunden sind, für eine der folgenden Gruppierungen steht

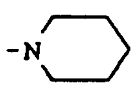 ; 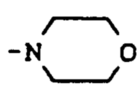 ; 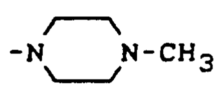 ; 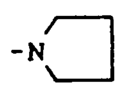 ;

wobei die Verbindungen ausgenommen sind, in denen $R^4$ und $R^5$ gleichzeitig für Wasserstoff stehen, Z für Alkoxy oder Alkylthio steht und

a) $R^1$ und $R^2$ gleichzeitig für Wasserstoff stehen oder

b) einer der Reste $R^1$ oder $R^2$ für Wasserstoff steht und der andere Rest $R^2$ bzw. $R^1$ in der ortho oder meta-Position des Phenyl für Halogen, Methyl oder Trifluormethyl steht,

und wobei die Verbindungen
2,6-Bis(4-methoxyphenyl)-4-methylthio-pyridin,
4-(1-Methylethyl)-2,6-diphenyl-pyridin,
4-Methyl-2,6-diphenyl-pyridin,
2,6-Bis(p-chlorphenyl)-4-picolin und
4-Ethyl-2,6-diphenyl-pyridin
ausgenommen sind.

Im einzelnen seien außer den bei den Herstellungsbeispielen genannten Verbindungen die folgenden 2,6-Diarylpyridin-Derivate der allgemeinen Formel (I) genannt:

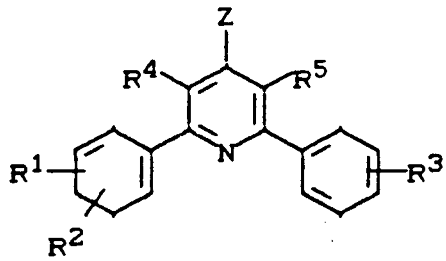

(I)

Tabelle 1

| $R^1$ | $R^2$ | $R^3$ | $R^4$ | Z | $R^5$ |
|---|---|---|---|---|---|
| 2-F | 4-F | $3\text{-}CF_3$ | H | $-OCH_3$ | H |
| 2-F | 4-F | $3\text{-}CF_3$ | H | $-OC_3H_7\text{-}n$ | H |
| 2-F | 4-F | $3\text{-}CF_3$ | H | $-OCH_2\text{-}CH{=}CH_2$ | H |
| 2-F | 4-F | $3\text{-}CF_3$ | H | $-OCH_2\text{-}C{\equiv}CH$ | H |
| 2-F | 4-F | $3\text{-}CF_3$ | H | $-OC_4H_9\text{-}n$ | H |
| 2-F | 4-F | $3\text{-}CF_3$ | H | $-OC_3H_7\text{-}i$ | H |
| 2-F | 4-F | $3\text{-}CF_3$ | H | $-OC_2H_5$ | $CH_3$ |
| 2-F | 4-F | $3\text{-}CF_3$ | $CH_3$ | $-OC_2H_5$ | H |
| 2-F | 4-F | $3\text{-}CF_3$ | H | $-SC_2H_5$ | H |
| 2-F | 4-F | $3\text{-}CF_3$ | H | $-SC_3H_7\text{-}n$ | H |
| 2-F | 4-F | $3\text{-}CF_3$ | H | $-N(CH_3)_2$ | H |
| 2-F | 4-F | $3\text{-}CF_3$ | H | $-N(C_2H_5)_2$ | H |
| 2-F | 4-F | $3\text{-}CF_3$ | H | $-N(C_3H_7\text{-}n)_n$ | H |
| 2-F | 4-F | $3\text{-}CF_3$ | H | 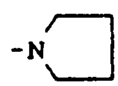 | H |
| 2-F | 4-F | $3\text{-}CF_3$ | H | $-OCH_3$ | $-C_2H_5$ |
| 2-F | 4-F | $3\text{-}CF_3$ | $-C_2H_5$ | $-OCH_3$ | H |
| 2-F | 4-F | $3\text{-}CF_3$ | H | $-NHCH_3$ | H |
| 2-F | 4-F | $3\text{-}CF_3$ | H | $-NHC_2H_5$ | H |
| 2-F | 4-F | $3\text{-}CF_3$ | H | $-NHC_3H_7\text{-}n$ | H |
| 2-F | 4-F | $3\text{-}CF_3$ | H | $-NHC_3H_7\text{-}i$ | $CH_3$ |

Tabelle 1 (Fortsetzung)

| $R^1$ | $R^2$ | $R^3$ | $R^4$ | Z | $R^5$ |
|---|---|---|---|---|---|
| 2-F | 4-F | $3-CF_3$ | $CH_3$ | $-SCH_3$ | $CH_3$ |
| 2-F | 4-F | $3-CF_3$ | $CH_3$ | $-SC_2H_5$ | $CH_3$ |
| 2-F | 4-F | $3-CF_3$ | $CH_3$ | $-OCH_3$ | $CH_3$ |
| 2-F | 4-F | $3-CF_3$ | $CH_3$ | $-OC_2H_5$ | $CH_3$ |
| 2-F | 4-F | 3-Cl | H | $-SCH_3$ | H |
| 2-F | 4-F | 3-Cl | H | $-SC_2H_5$ | H |
| 2-F | 4-F | 3-Cl | H | $-OCH_3$ | H |
| 2-F | 4-F | 3-Cl | H | $-OC_2H_5$ | H |
| 2-F | 4-F | 3-Cl | H | 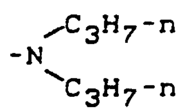 | H |
| 2-F | 4-F | 3-Cl | H | 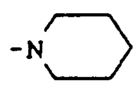 | H |
| 2-F | 4-F | 3-Cl | H | 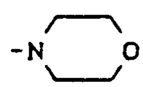 | H |
| 2-F | 4-F | 3-Cl | H | $-SCH_3$ | $CH_3$ |
| 2-F | 4-F | 3-Cl | H | $-SC_2H_5$ | $CH_3$ |
| 2-F | 4-F | 3-Cl | H | $-OCH_3$ | $CH_3$ |
| 2-F | 4-F | 3-Cl | H | $-OC_2H_5$ | $CH_3$ |
| 2-F | 4-F | 3-Cl | $CH_3$ | $-OC_3H_7$-n | H |
| 2-F | 4-F | 3-Cl | $CH_3$ | $-OC_2H_5$ | H |
| 2-F | 4-F | 3-Cl | $CH_3$ | $-OCH_3$ | $CH_3$ |

Tabelle 1 (Fortsetzung)

| $R^1$ | $R^2$ | $R^3$ | $R^4$ | Z | $R^5$ |
|---|---|---|---|---|---|
| 2-Cl | 4-Cl | $3-CF_3$ | H | $-SCH_3$ | H |
| 2-Cl | 4-Cl | $3-CF_3$ | H | $-SC_2H_5$ | H |
| 2-Cl | 4-Cl | $3-CF_3$ | H | $-SC_3H_7-i$ | $CH_3$ |
| 2-Cl | 4-Cl | $3-CF_3$ | H | $-OCH_3$ | H |
| 2-Cl | 4-Cl | $3-CF_3$ | H | $-OC_2H_5$ | H |
| 2-Cl | 4-Cl | $3-CF_3$ | H | $-OC_3H_7-n$ | H |
| 2-Cl | 4-Cl | $3-CF_3$ | $CH_3$ | $-OC_3H_5$ | H |
| 2-Cl | 4-Cl | $3-CF_3$ | H | $-OCH_2-C\equiv CH$ | H |
| 2-Cl | 4-Cl | 3-Cl | H | $-SCH_3$ | H |
| 2-Cl | 4-Cl | 3-Cl | H | $-SC_2H_5$ | H |
| 2-Cl | 4-Cl | 3-Cl | H | $-OCH_3$ | H |
| 2-Cl | 4-Cl | 3-Cl | H | $-OC_2H_5$ | H |
| 2-Cl | 4-Cl | 3-Cl | H | $-NHC_2H_5$ | H |
| 2-Cl | 4-Cl | 3-Cl | H | $-NHCH_3$ | H |
| 2-F | 2-F | $3-CH_3$ | H | $-SCH_3$ | H |
| 2-F | 2-F | $3-CH_3$ | H | $-SC_2H_5$ | H |
| 2-F | 2-F | $3-CH_3$ | H | $-SCH_2-CH=CH_2$ | H |
| 2-F | 2-F | $3-CH_3$ | H | $-OC_2H_5$ | H |
| 2-F | 2-F | 3-F | H | $-SCH_3$ | H |
| 2-F | 2-F | 3-F | H | $-SC_2H_5$ | H |
| 2-F | 2-F | 3-F | H | $-OCH_3$ | H |

Tabelle 1 (Fortsetzung)

| $R^1$ | $R^2$ | $R^3$ | $R^4$ | Z | $R^5$ |
|---|---|---|---|---|---|
| 2-F | 2-F | 3-F | H | $-OC_2H_5$ | H |
| 2-F | 2-F | 3-F | H | $-OCH_2-CH=CH_2$ | H |
| 2-F | 2-F | 3-F | H | $-OCH_2-C\equiv CH$ | H |
| 2-F | 2-F | 3-Br | H | $-SCH_3$ | H |
| 2-F | 2-F | 3-Br | H | $-OC_2H_5$ | H |
| 2-F | 2-F | 3-Br | H | $-OCH_3$ | $CH_3$ |
| $2-CH_3$ | $4-CH_3$ | $3-CF_3$ | H | $-SCH_3$ | H |
| $2-CH_3$ | $4-CH_3$ | $3-CF_3$ | H | $-OCH_3$ | H |
| $2-CH_3$ | $4-CH_3$ | $3-CF_3$ | $CH_3$ | $-SCH_3$ | H |
| $2-CH_3$ | $4-CH_3$ | $3-CF_3$ | $CH_3$ | $-OCH_3$ | H |
| $2-CH_3$ | $4-CH_3$ | $3-CF_3$ | H | $-OC_2H_5$ | H |
| $2-CH_3$ | $4-CH_3$ | $3-CF_3$ | H | $-OCH_2-CH=CH_2$ | H |
| $2-CH_3$ | $4-CH_3$ | $3-CF_3$ | H | $-OC_3H_7-n$ | H |
| $3-CF_3$ | $5-CF_3$ | H | H | $-SCH_3$ | H |
| $3-CF_3$ | $5-CF_3$ | H | H | $-SC_2H_5$ | H |
| $3-CF_3$ | $5-CF_3$ | H | H | $-SCH_2-CH=CH_2$ | H |
| $3-CF_3$ | $5-CF_3$ | H | H | $-OCH_2-CH=CH_2$ | H |
| $3-CF_3$ | $5-CF_3$ | H | H | $-OC_2H_5$ | $CH_3$ |
| $3-CF_3$ | $5-CF_3$ | 2-F | H | $-SCH_3$ | H |
| $3-CF_3$ | $5-CF_3$ | 2-F | H | $-OCH_3$ | H |
| $3-CF_3$ | $5-CF_3$ | 2-F | H | $-OC_2H_5$ | H |

Tabelle 1 (Fortsetzung)

| $R^1$ | $R^2$ | $R^3$ | $R^4$ | Z | $R^5$ |
|---|---|---|---|---|---|
| 3-$CF_3$ | 5-$CF_3$ | 2-F | H | -$OC_2H_5$ | $CH_3$ |
| 3-$CF_3$ | 5-$CF_3$ | 2-Cl | H | -$SCH_3$ | H |
| 3-$CF_3$ | 5-$CF_3$ | 2-Cl | H | -$OC_2H_5$ | H |
| 3-$CF_3$ | 5-$CF_3$ | 2-Cl | H | -$OCH_2$-CH=$CH_2$ | H |
| 3-$CF_3$ | 5-$CF_3$ | 2-$CF_3$ | H | -$SCH_3$ | H |
| 3-$CF_3$ | 5-$CF_3$ | 2-$CF_3$ | H | -$OCH_3$ | H |
| 3-$CF_3$ | 5-$CF_3$ | 2-$CF_3$ | H | -$OC_2H_5$ | H |
| 3-$CF_3$ | 5-$CF_3$ | 2-$CF_3$ | H | -N($C_3H_7$-n)$_2$ | H |
| 3-$CF_3$ | 5-$CF_3$ | 3-F | H | -$SCH_3$ | H |
| 3-$CF_3$ | 5-$CF_3$ | 3-F | H | -$OCH_3$ | H |
| 3-$CF_3$ | 5-$CF_3$ | 3-F | H | -$OC_2H_5$ | H |
| 3-$CF_3$ | 5-$CF_3$ | 4-F | H | -$SCH_3$ | H |
| 3-$CF_3$ | 5-$CF_3$ | 4-F | H | -$OC_2H_5$ | H |
| 3-$CF_3$ | 5-$CF_3$ | 4-F | H | -$OCH_2$-CH=$CH_2$ | H |
| 3-$C_2H_5$ | H | H | H | -$OCH_3$ | $C_2H_5$ |
| 3-$C_2H_5$ | H | H | H | -$OC_2H_5$ | $C_2H_5$ |
| 3-$C_2H_5$ | H | H | H | -$OCH_3$ | $CH_3$ |
| 3-$C_2H_5$ | H | H | H | -$OCH_2$-CH=$CH_2$ | H |
| 2-$CH_3O$ | H | 3-Cl | H | -$OCH_3$ | H |
| 2-$CH_3O$ | H | 3-Cl | H | -$OC_2H_5$ | $CH_3$ |
| 2-$CH_3O$ | H | 3-$CF_3$ | H | -$OCH_2$-C≡CH | H |

Tabelle 1 (Fortsetzung)

| $R^1$ | $R^2$ | $R^3$ | $R^4$ | Z | $R^5$ |
|---|---|---|---|---|---|
| 3-Cl | H | H | $CH_3$ | $-SCH_3$ | $-CH_3$ |
| 3-Cl | H | H | $CH_3$ | $-OCH_3$ | $-CH_3$ |
| 3-Cl | H | H | $CH_3$ | $-OC_2H_5$ | $-CH_3$ |
| 3-Cl | H | 4-Cl | $CH_3$ | $-SCH_3$ | $-CH_3$ |
| 3-Cl | H | 4-Cl | $CH_3$ | $-OC_2H_5$ | $-CH_3$ |
| 3-Cl | H | 4-F | $CH_3$ | $-SCH_3$ | $-CH_3$ |
| 3-Cl | H | 4-F | $CH_3$ | $-OC_2H_5$ | $-CH_3$ |
| $3-CF_3$ | H | H | H | $-CH_3$ | H |
| $3-CF_3$ | H | H | $CH_3$ | $-CH_3$ | H |
| $3-CF_3$ | H | 4-Cl | H | $-CH_3$ | H |
| $3-CF_3$ | H | 4-F | H | $-CH_3$ | H |
| $3-CF_3$ | H | $4-CH_3$ | H | $-CH_3$ | H |
| $3-CF_3$ | H | 2-Cl | H | $-CH_3$ | H |
| 3-Cl | H | H | H | $-CH_3$ | H |
| 3-Cl | H | H | $CH_3$ | $-CH_3$ | H |
| 3-Cl | H | $2-CH_3$ | H | $-CH_3$ | H |
| 3-Cl | H | 2-F | H | $-CH_3$ | H |
| 3-Cl | H | 4-F | H | $-CH_3$ | H |
| 3-Cl | H | 4-Cl | H | $-CH_3$ | H |
| 3-Cl | H | 4-Cl | $CH_3$ | $-CH_3$ | H |
| $3-CH_3$ | H | H | H | $-CH_3$ | H |

Tabelle 1 (Fortsetzung)

| $R^1$ | $R^2$ | $R^3$ | $R^4$ | Z | $R^5$ |
|---|---|---|---|---|---|
| $3-C_2H_5$ | H | H | H | $-CH_3$ | H |
| $2-CH_3O$ | H | H | H | $-CH_3$ | H |
| 2-F | 4-F | H | H | $-C_2H_5$ | H |
| 2-F | H | H | H | $-C_2H_5$ | H |
| $2-C_2H_5O$ | H | H | H | $-C_2H_5$ | H |
| 3-Cl | 5-Cl | H | H | $-C_2H_5$ | H |

Verwendet man beispielsweise 3-Methylthio-1-phenyl-5-(3-trifluorphenyl)-3-penten-1,5-dion und Ammoniumacetat als Ausgangsstoffe, so läßt sich der Reaktionsablauf des erfindungsgemäßen Verfahrens (a) durch das folgende Formelschema darstellen:

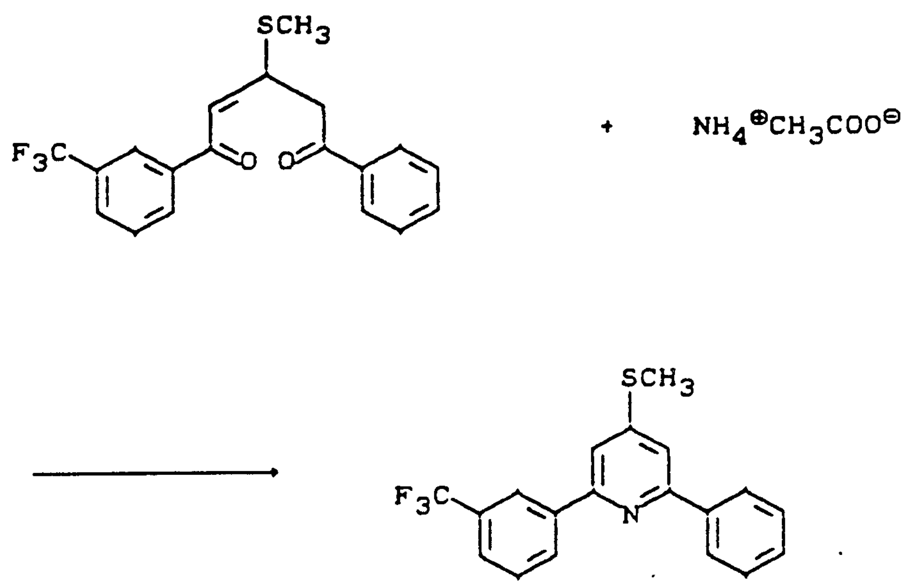


Verwendet man beispielsweise 2-(3-Chlorphenyl)-4-methylsulfonyl-6-(phenyl)-pyridin und Kaliumethylat als Ausgangsstoffe, so läßt sich der Reaktionsablauf des erfindungsgemäßen Verfahrens (b-α) durch das folgende Formelschema darstellen:

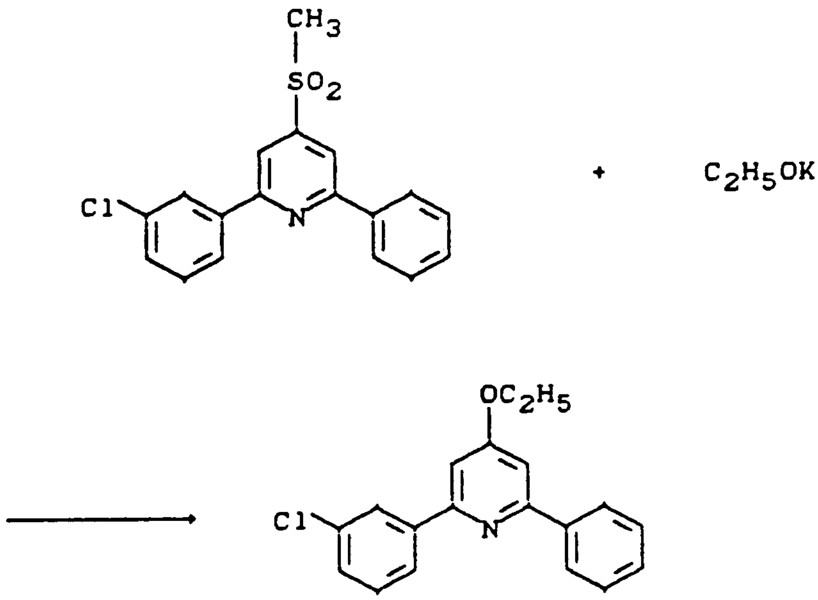

Verwendet man beispielsweise 2-(3-Chlorphenyl)-4-methylsulfonyl-6-(phenyl)-pyridin und Diethylamin als Ausgangsstoffe, so läßt sich das erfindungsgemäße Verfahren (b-β) durch das folgende Formelschema darstellen:

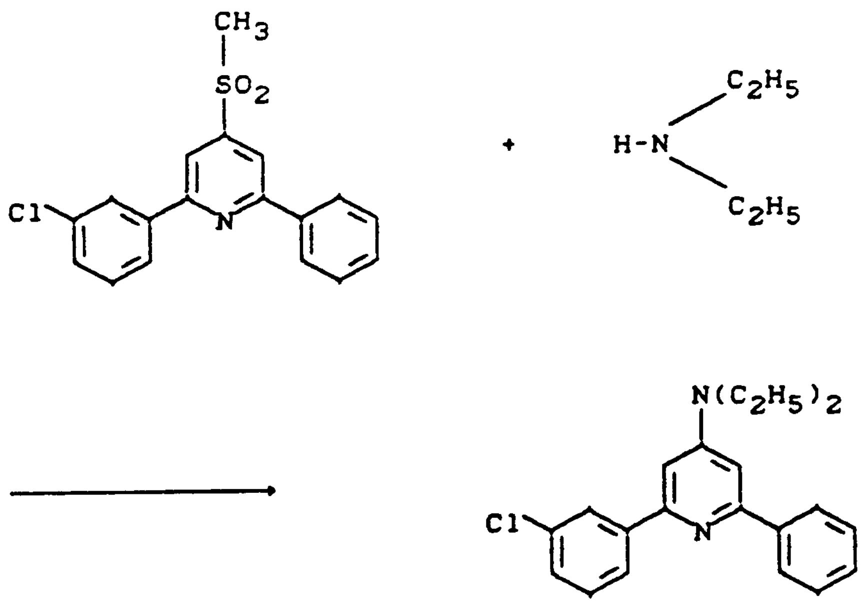

Verwendet man beispielsweise 2-(4-Chlorphenyl)-4-hydroxy-6-(phenyl)-pyridin und Phosphorylchlorid als Ausgangsstoffe, so läßt sich das erfindungsgemäße Verfahren (c) durch das folgende Formelschema darstellen:

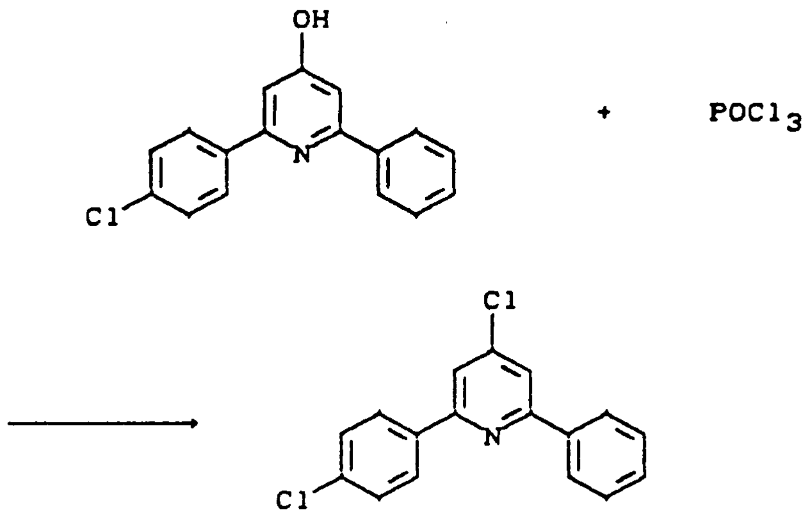


Verwendet man beispielsweise 2-(4-Chlorphenyl)-4-chlor-6-(phenyl)-pyridin und Malonsäurediethylester als Ausgangsstoffe, so läßt sich das erfindungsgemäße Verfahren (d) durch das folgende Formelschema darstellen:

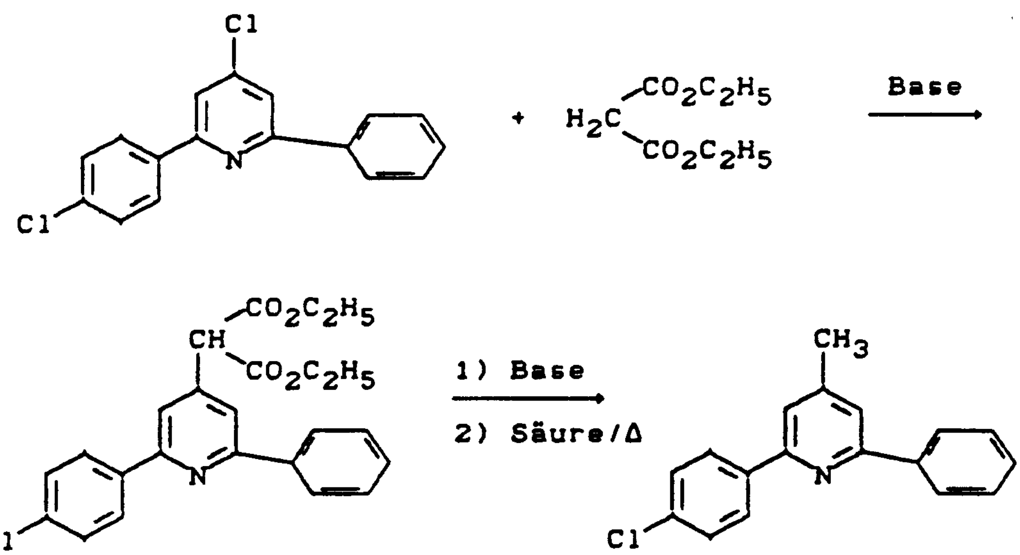


Die Verbindungen der Formel (II) und (IIa) sind teilweise bekannt. Sie lassen sich analog zu bekannten Verfahren herstellen, indem man beispielsweise entweder Ketone der Formel (VIII)

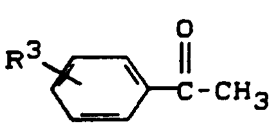

(VIII)

in welcher

$R^3$ die oben angegebene Bedeutung hat,

mit arylsubstituierten α-Oxoketendithioacetal-Derivaten der Formel (IX)

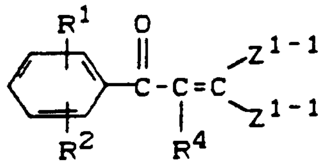

(IX)

in welcher
$R^1$, $R^2$, $R^4$ und $Z^{1-1}$ die oben angegebene Bedeutung haben,
oder indem man Ketone der Formel (X)

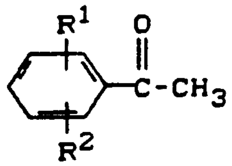

(X)

in welcher
$R^1$ und $R^2$ die oben angegebene Bedeutung haben,
mit arylsubstituierten α-Oxoketendithioacetal-Derivaten der Formel (XI)

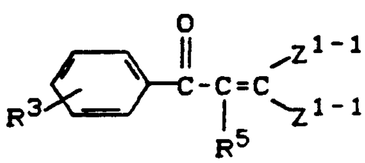

(XI)

in welcher
$Z^{1-1}$, $R^3$ und $R^5$ die oben angegebene Bedeutung haben,
gegebenenfalls in Gegenwart eines Verdünnungsmittels wie Diethylether, Dioxan, Diethylenglykoldimethylether oder Tetrahydrofuran und gegebenenfalls in Gegenwart einer Base wie beispielsweise Natriumhydrid oder Kalium-tert-butylat bei Temperaturen zwischen 0 °C und +50 °C umsetzt (vergl. EP-A 263 958).

Arylsubstituierte α-Oxoketendithioacetal-Derivate der Formel (IX) sowie arylsubstituierte α-Oxoketendithioacetal-Derivate der Formel (XI) sind ebenfalls teilweise bekannt und/oder können nach bekannten Verfahren hergestellt werden, indem man beispielsweise Acetylverbindungen der Formel (XII)

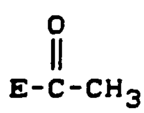

(XII)

in welcher
E für die Bedeutung von

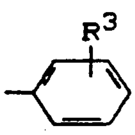

oder

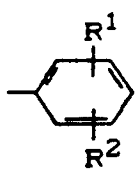

steht,
wobei
$R^1$, $R^2$ und $R^3$ die oben angegebene Bedeutung haben,
mit Schwefelkohlenstoff, gegebenenfalls in Gegenwart eines Verdünnungsmittels wie N,N-Dimethylformamid

oder Dioxan oder Tetrahydrofuran in Gegenwart einer Base wie beispielsweise Kalium-tert-butylat oder Natriumhydrid und anschließend mit einem Alkylierungsreagenz der Formel (XIII)

$Z^{1-1}$-$Y^1$ (XIII)

in welcher

$Z^{1-1}$ die oben angegebene Bedeutung hat, und

$Y^1$ für Chlor, Brom oder Iod steht,

bei Temperaturen zwischen 0°C und +50°C umsetzt (vergleiche J. Chem. Soc., Perkin Trans. 1 (6), 549-553 (1978), EP-A 263 958).

Acetophenon-Derivate der Formel (X), Ketone der Formel (VIII), Acetylverbindungen der Formel (XII) sowie Alkylierungsreagenzien der Formel (XIII) sind allgemein bekannte Verbindungen der organischen Chemie.

Die beim erfindungsgemäßen Verfahren (b) zur Herstellung von Verbindungen der Formel (Ib) als Ausgangsstoffe benötigten 4-Sulfonylalkyl-2,6-Diarylpyridin-Derivate sind durch die Formel (III) allgemein definiert.

In Formel (III) steht $R^9$ vorzugsweise für geradkettiges oder verzweigtes Alkyl mit 1 bis 4 Kohlenstoffatomen, insbesondere für Methyl oder Ethyl.

Die 4-Sulfonylalkyl-2,6-Diarylpyridin-Derivate der Formel (III) sind neu. Sie lassen sich jedoch analog zu bekannten Verfahren herstellen (vergl. EP-A 263 958), indem man 2-Aryl-6-hetarylpyridin-Derivate der Formel (Ia)

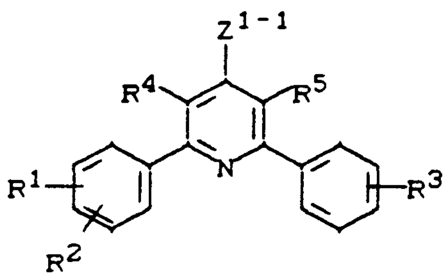

(Ia)

in welcher

$R^1$, $R^2$, $R^3$, $R^4$, $R^5$ und $Z^{1-1}$ die oben angegebene Bedeutung haben,

mit einem Oxidationsmittel wie beispielsweise Metachlorperbenzoesäure oder Wasserstoffperoxid/Ameisensäure, gegebenenfalls in Gegenwart eines Katalysators wie beispielsweise Ammoniummolybdat, gegebenenfalls in Gegenwart eines Verdünnungsmittels wie beispielsweise Hexan, Heptan, Petrolether, Benzol, Toluol, Chlorbenzol, Diethylether, Dioxan, Tetrahydrofuran, Aceton, Methanol, Ethanol, Wasser oder Mischungen dieser Verdünnungsmittel, bei Temperaturen zwischen 0°C und +130°C umsetzt.

In Formel (IV) steht M für ein Alkalimetallkation, vorzugsweise für ein Natrium- oder Kaliumkation.

Die Verbindungen der Formel (IV) sowie die Amine der Formel (V) sind allgemein bekannte Verbindungen der organischen Chemie.

Die 4-Hydroxy-2,6-Diarylpyridin-Derivate der Formel (VI) sind neu. Sie lassen sich jedoch analog zu bekannten Verfahren (vergl. EP-A 263 958) herstellen, indem man 4-Sulfonylalkyl-2,6-Diarylpyridin-Derivate der Formel (III)

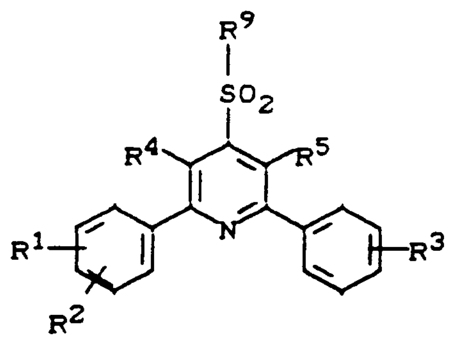

(III)

in welcher

$R^1$, $R^2$, $R^3$, $R^4$, $R^5$ und $R^9$ die oben angegebene Bedeutung haben,

mit Alkalihydroxid wie beispielsweise Natrium- oder Kaliumhydroxid, gegebenenfalls in Gegenwart eines Verdünnungsmittels wie beispielsweise Methanol, Ethanol, Wasser, Dimethylformamid, Acetonitril, Tetrahydrofuran oder Dioxan bei Temperaturen zwischen 0° C und +160° C umsetzt.

In Formel (VII) steht

$Z^{3-1}$ vorzugsweise für Wasserstoff, Alkyl mit 1 bis 8 Kohlenstoffatomen, Alkenyl mit 2 bis 6 Kohlenstoffatomen, Alkinyl mit 2 bis 6 Kohlenstoffatomen oder Trimethylsilylalkinyl mit 2 bis 6 Kohlenstoffatomen im Alkinylteil, besonders bevorzugt für Wasserstoff, jeweils Alkyl mit 1 bis 6 Kohlenstoffatomen, Alkenyl mit 2 bis 4 Kohlenstoffatomen, Alkinyl mit 2 bis 4 Kohlenstoffatomen oder Trimethylsilylalkinyl mit 2 bis 4 Kohlenstoffatomen im Alkinylteil.

Insbesondere steht $Z^{3-1}$ für Wasserstoff, Methyl, Ethyl, n-Propyl, i-Propyl, n-Butyl, s-Butyl, i-Butyl, t-Butyl, Methoxymethyl, Ethoxymethyl, Methoxycarbonylmethyl, Ethoxycarbonylmethyl, Allyl, Propargyl oder 2-Trimethylsilylethinyl.

$Z^3$ in Formel (Id) steht mit Ausnahme von Wasserstoff für die entsprechenden Reste in $Z^{3-1}$.

In Formel (VII) steht $R^{10}$ vorzugsweise für Methyl oder Ethyl.

Diester-Derivate der Formel (VII) sind allgemein bekannte Verbindungen der organischen Chemie.

Das erfindungsgemäße Verfahren (a) wird vorzugsweise unter Verwendung von Verdünnungsmitteln durchgeführt. Als Verdünnungsmittel kommen dabei praktisch alle inerten organischen Lösungsmittel infrage. Hierzu gehören Ether wie Diethylether, Diisopropylether, Dioxan, Tetrahydrofuran und Diethylenglykoldimethylether; Carbonsäuren wie Ameisensäure und Essigsäure; Alkohole wie Methanol, Ethanol, Isopropanol, tert-Butanol, Octanol, Cyclohexanol, Diethylenglykol und Glycerin; Amide wie Formamid, N,N-Dimethylsulfoxid und Sulfolan sowie Mischungen der oben genannten Verdünnungsmittel. Vorzugsweise verwendet man Tetrahydrofuran oder N,N-Dimethylformamid.

Die Reaktionstemperaturen können bei dem erfindungsgemäßen Verfahren (a) in einem größeren Bereich variiert werden. Im allgemeinen arbeitet man bei Temperaturen zwischen 0 °C und +100 °C, vorzugsweise bei Temperaturen zwischen 0 °C und +50 °C.

Zur Durchführung des erfindungsgemäßen Verfahrens (a) setzt man pro Mol an 1,5-Dioxo-Verbindung der Formel (II) oder (IIa) im allgemeinen 1 bis 20 Mol, vorzugsweise 5 bis 10 Mol an Ammoniumsalz (beispielsweise Ammoniumacetat oder Ammoniumchlorid) oder Ammoniate ein.

Dabei ist es auch möglich, die als Ausgangsverbindung zur Durchführung des erfindungsgemäßen Verfahrens (a) benötigten 1,5-Dioxo-Verbindungen der Formel (II) bzw. (IIa) in einer vorgelagerten Reaktion direkt im Reaktionsgefäß herzustellen und direkt aus dem Reaktionsgemisch heraus ohne Isolierung weiter umzusetzen ("Eintopfverfahren").

Die Reaktionsdurchführung und Isolierung der Reaktionsprodukte erfolgt bei allen Verfahren nach allgemein üblichen Methoden.

Das erfindungsgemäße Verfahren (b-α) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Chlorbenzol und o-Dichlorbenzol, Ether wie Diethyl- und Diisopropylether, Glykoldimethylether und Diglykoldimethylether, Tetrahydrofuran und Dioxan, Nitrile wie z.B. Acetonitril und Isobutyronitril; Alkohole wie Methanol, Ethanol, Isopropanol und tert-Butanol; tertiäre Amine wie Pyridin, Triethylamin, N,N-Diethylanilin, Tributylamin und N-Methylmorpholin; Amide wie Formamid, N,N-Dimethylformamid und Acetamid;

Das erfindungsgemäße Verfahren wird bei Temperaturen zwischen 0 °C und +150 °C, vorzugsweise bei

Temperaturen zwischen 0 °C und +120 °C durchgeführt.

Zur Durchführung des erfindungsgemäßen Verfahrens (b-α) setzt man pro Mol an 4-Sulfonylalkyl-2,6-Diarylpyridin-Derivat der Formel (III) im allgemeinen 1 bis 15 Mol, vorzugsweise 1 bis 10 Mol an Verbindung der Formel (IV) ein.

Das erfindungsgemäße Verfahren (b-β) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon, Alkohole wie z.B. Methanol, Ethanol, Isopropanol, tert-Butanol, Carbonsäuren wie z.B. Ameisensäure und Essigsäure, Wasser sowie Dimethylsulfoxid, Tetramethylensulfon und Hexamethylphosphorsäuretriamid oder Gemische der oben genannten Verdünnungsmittel.
außerdem Dimethylsulfoxid und Sulfolan sowie Mischungen der oben genannten Verdünnungsmittel.

Die Reaktionstemperaturen können bei dem erfindungsgemäßen Verfahren (b-α) in einem größeren Bereich variiert werden. Im allgemeinen arbeitet man bei Temperaturen Die Reaktionstemperaturen können bei dem erfindungsgemäßen Verfahren (b-β) in einem größeren Bereich variiert werden. Im allgemeinen arbeitet man bei Temperaturen zwischen 0 °C und +180 °C, vorzugsweise bei Temperaturen zwischen +20 °C und +120 °C.

Zur Durchführung des erfindungsgemäßen Verfahrens (b-β) setzt man pro Mol an 4-Sulfonylalkyl-2,6-Diarylpyridin-Derivat der Formel (III) im allgemeinen 1 bis 100 Mol, vorzugsweise 1 bis 10 Mol an Amin der Formel (V) oder (Va) ein.

Das erfindungsgemäße Verfahren (b-β) wird vorzugsweise unter Normaldruck durchgeführt, es ist jedoch auch möglich unter erhöhtem Druck zu arbeiten.

Das erfindungsgemäße Verfahren (c) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (c) wird unter Verwendung von Halogenierungsreagenzien durchgeführt. Vorzugsweise verwendet man Thionylchlorid, Phosgen, Phosphorylchlorid, Phosphorpentachlorid, Phosphorylbromid oder Phosphortribromid.

Die Reaktionstemperaturen können bei dem erfindungsgemäßen Verfahren (c) in einem größeren Bereich variiert werden. Im allgemeinen arbeitet man bei Temperaturen zwischen 0 °C und +200 °C, vorzugsweise bei Temperaturen zwischen +50 °C und +150 °C.

Zur Durchführung des erfindungsgemäßen Verfahrens (c) setzt man pro Mol an 4-Hydroxy-2,6-Diarylpyridin-Derivat der Formel (VI) im allgemeinen 1 bis 50 Mol, vorzugsweise 1 bis 10 Mol an Halogenierungsreagenz ein.

Das erfindungsgemäße Verfahren (d) wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Dimethylsulfoxid und Sulfolan sowie Gemische dieser Verdünnungsmittel.

Die Reaktionstemperaturen können beim ersten Reaktionsschritt des erfindungsgemäßen Verfahren (d) in einem größeren Bereich variiert werden. Im allgemeinen arbeitet man bei Temperaturen zwischen -10 °C und +180 °C, vorzugsweise bei Temperaturen zwischen 0 °C und +150 °C.

Der erste Reaktionsschritt des erfindungsgemäßen Verfahrens (d) wird in Gegenwart von Basen

durchgeführt. Vorzugsweise verwendet man Alkalimetallhydride wie beispielsweise Natriumhydrid, Alkyllithium wie n-Butyllithium, Lithiumdialkylamide wie z.B. Lithiumdiisopropylamid (LDA), Alkalimetallalkoholate wie z.B. Natriummethanolat und Natriumethanolat und Metallhydroxide wie z.B. Natriumhydroxid.

Zur Durchführung des ersten Reaktionsschrittes des erfindungsgemäßen Verfahrens (d) setzt man pro Mol an Verbindung der Formel (Ic) im allgemeinen 0,5 bis 2 Mol, vorzugsweise 1 bis 1,5 Mol an Diester-Derivat der Formel (VII) sowie im allgemeinen 1 bis 3 Mol, vorzugsweise 1 bis 2 Mol an Base ein.

Die Reaktionstemperaturen können beim zweiten Reaktionsschritt des erfindungsgemäßen Verfahrens (d) in einem größeren Bereich variiert werden. Im allgemeinen arbeitet man bei Temperaturen zwischen 0 °C und +150 °C, vorzugsweise bei Temperaturen zwischen +10 °C und +100 °C.

Der zweite Reaktionsschritt des erfindungsgemäßen Verfahrens (d) wird in Gegenwart von Basen durchgeführt. Vorzugsweise verwendet man Alkalimetallhydroxide wie z.B. Natriumhydroxid und Alkalimetallcarbonate wie z.B. Natriumcarbonat.

Zur Durchführung des zweiten Reaktionsschrittes des erfindungsgemäßen Verfahrens (d) setzt man pro Mol an verwendeter Verbindung der Formel (Ic) im allgemeinen 1 bis 6 Mol, vorzugsweise 2,1 bis 5 Mol an Base ein.

Die Reaktionstemperaturen können bei der Decarboxylierungsreaktion des erfindungsgemäßen Verfahrens (d) in einem größeren Bereich variiert werden. Im allgemeinen arbeitet man bei Temperaturen zwischen 0 °C und +200 °C, vorzugsweise bei Temperaturen zwischen +20 °C und +150 °C.

Die Decarboxylierungsreaktion des erfindungsgemäßen Verfahrens (d) wird in Gegenwart von Säuren durchgeführt. Vorzugsweise verwendet man anorgansiche Säuren wie z.B. Schwefelsäure oder Chlorwasserstoffsäure und organische Säuren wie z.B. Essigsäure.

Zur Durchführung der Decarboxylierungsreaktion des erfindungsgemäßen Verfahrens (d) setzt man pro Mol an verwendeter Verbindung der Formel (Ic) im allgemeinen 1 bis 10 Mol, vorzugsweise 2,5 bis 6 Mol an Säure ein.

Die Durchführung des erfindungsgemäßen Verfahrens (d) erfolgt nach üblichen Methoden. Zur Aufarbeitung wird die Reaktionsmischung neutralisiert, konzentriert und extrahiert. Die Reinigung des Produktes erfolgt durch Chromatographie oder Umkristallisation.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei eignen sich die erfindungsgemäßen Wirkstoffe der Formel (I) besonders gut zur selektiven Bekämpfung von dikotylen Unkräutern in monokotylen Kulturen, wie beispielsweise Weizen, im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Weiterhin kommen 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxybenzonitril (BROMOXYNIL); 2-Chlor-N- [(4-methoxy-6-methyl-1,3,5-triazin-2-yl)-amino]-carbonyl -benzolsulfonamid (CHLORSULFURON); 2-[4-(2,4-Dichlorphenoxy)-phenoxy]-propionsäure, deren Methyl- oder deren Ethylester (DICLOFOPMETHYL); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenylpyridazin-4-yl)-S-octylthiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sul fonyl]-thiophen-2-carbonsäure-methylester (THIAMETURON) in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln

ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiel

Beispiel 1

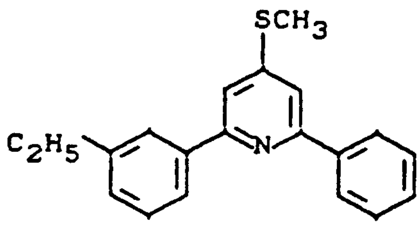

3 g (0,02 mol) 3-Ethylacetophenon werden in 100 ml trockenem Tetrahydrofuran mit 5,2 g (0,046 mol) Kalium-tert.-butylat 30 Minuten bei Raumtemperatur gerührt. Danach werden 4,5 g (0,02 mol) 3,3-Bis-(methylthio)-1-phenyl-2-propen-1-on zugesetzt und weitere 60 Minuten bei Raumtemperatur gerührt. Nach Zugabe von 15 g Ammoniumacetat und 100 ml Eisessig wird 3 Stunden bei Rückfluß erhitzt, wobei das Tetrahydrofuran über einen Wasserabscheider abdestilliert wird. Das Reaktionsgemisch wird auf Raumtemperatur abgekühlt, mit Natronlauge neutralisiert, mit Essigester ausgeschüttelt, mit Natriumsulfat getrocknet und unter vermindertem Druck eingeengt.

Man erhält 4,0 g (66 % der Theorie) 4-Methylthio-2-(3-ethyl-phenyl)-6-phenyl-pyridin als Öl.
($^1$H-NMR ($CDCl_3$) $\delta$ = 2,7 (q, 2H), 2,6 (s, 3H), 1,3 (t, 3H).

Beispiel 2

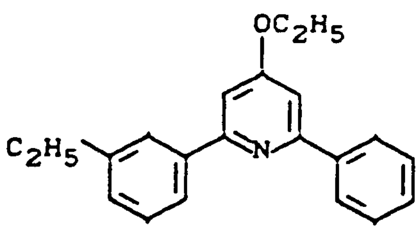

1,5 g (0,004 mol) 4-Methylsulfonyl-2-(3-ethyl-phenyl)-6-phenyl-pyridin werden mit 1,1 g (0,013 mol) Kaliumethylat in 30 ml trockenem Ethanol versetzt, die Mischung wird über Nacht unter Rückfluß zum Sieden erhitzt und dann nach dem Abkühlen auf Raumtemperatur unter vermindertem Druck eingeengt. Danach wird der Rückstand mit Methyl-tert.-butylether aufgenommen, mit Wasser gewaschen, mit Natriumsulfat getrocknet und unter vermindertem Druck eingeengt.

Man erhält 0,9 g (67 % der Theorie) 4-Ethoxy-2-(3-ethylphenyl)-6-phenyl-pyridin als Öl.

$^1H$-NMR ($CDCl_3$) $\delta$ = 4,2 (q, 2H), 2,7 (q, 2H), 1,5 (t, 3H), 1,3 (t, 3H).

Analog zu den Herstellungsbeispielen 1 und 2 und gemäß den allgemeinen Angaben zu den erfindungsgemäßen Verfahren erhält man die in Tabelle 2 aufgeführten Verbindungen der allgemeinen Formel (I)

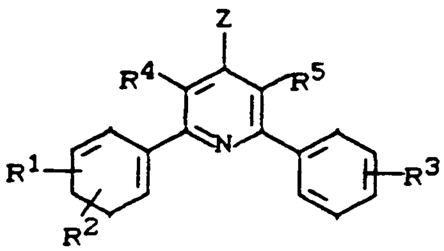

(I)

Tabelle 2

| Bsp.-Nr. | $R^1$ | $R^2$ | $R^3$ | $R^4$ | $R^5$ | Z | phys. Konstante ($^1$H-NMR* bzw. Schmelzpunkt) |
|---|---|---|---|---|---|---|---|
| 3 | 3-$C_2H_5$ | H | 4-Cl | H | H | -$SCH_3$ | 2.6 (s, 3H) |
| 4 | 2-$OCH_3$ | H | 4-Cl | H | H | -$SCH_3$ | 3.9 (s, 3H) |
| 5 | 2-$OCH_3$ | H | 3-Cl | H | H | -$SCH_3$ | 82° C |
| 6 | 3-$C_2H_5$ | H | 3-Cl | H | H | -$SCH_3$ | 2.6 (s, 3H) |
| 7 | 2-$OCH_3$ | H | 3-$CF_3$ | H | H | -$SCH_3$ | 3.9 (s, 3H) |
| 8 | 3-$C_2H_5$ | H | 3-$CF_3$ | H | H | -$SCH_3$ | 2.6 (s, 3H) |
| 9 | 2-$OCH_3$ | H | 3-$CF_3$ | H | H | -$OC_2H_5$ | 3.9 (s, 3H) |
| 10 | 3-$C_2H_5$ | H | 3-$CF_3$ | H | H | -$OC_2H_5$ | 2.7 (q, 2H) |
| 11 | 4-$C_2H_5$ | H | 3-$CF_3$ | H | H | -$SCH_3$ | 2.6 (s, 3H) |
| 12 | 4-$C_2H_5$ | H | 3-$CF_3$ | H | H | -$OC_2H_5$ | 2.7 (q, 2H) |
| 13 | 3-Cl | H | H | H | H | -$SCH_3$ | 102° C |
| 14 | 3-Cl | 5-Cl | 2-$CH_3$ | H | H | -$SCH_3$ | 106° C |
| 15 | 2-F | 4-F | 3-$CF_3$ | H | H | -$SCH_3$ | 82° C |
| 15 | 3-Cl | 5-Cl | 2-$OCH_3$ | H | H | -$SCH_3$ | 127° C |
| 17 | 3-Cl | 5-Cl | H | H | H | -$OC_2H_5$ | 101° C |
| 18 | 3-Cl | 5-Cl | 2-$CH_3$ | H | H | -$OC_2H_5$ | 100° C |
| 19 | 2-F | 4-F | 3-$CF_3$ | H | H | -$OC_2H_5$ | 71° C |
| 20 | 3-Cl | 5-Cl | 2-$OCH_3$ | H | H | -$OC_2H_5$ | 93° C |

**Tabelle 2** (Fortsetzung)

| Bsp.-Nr. | $R^1$ | $R^2$ | $R^3$ | $R^4$ | $R^5$ | Z | phys. Konstante ($^1$H-NMR* bzw. Schmelzpunkt) |
|---|---|---|---|---|---|---|---|
| 21 | 3-Cl | H | H | $-CH_3$ | H | $-SCH_3$ | 104° C |
| 22 | 3-Cl | H | H | $-CH_3$ | H | $OC_2H_5$ | 2.2 (s. 3H) |
| 23 | 2-F | 4-F | 2-Cl | H | H | $-SCH_3$ | 108° C |
| 24 | 3-Cl | 5-Cl | $3-CF_3$ | H | H | $-SCH_3$ | 127° C |
| 25 | 2-F | 4-F | 3-Cl | H | H | $-SCH_3$ | 88° C |
| 26 | 2-F | 4-F | $3-CF_3$ | H | H | $-OCH_3$ | 4.0 (s, 3H) |
| 27 | 3-Cl | 5-Cl | 3-Cl | H | H | $-SCH_3$ | 131° C |
| 28 | 2-Cl | 4-Cl | 3-Cl | H | H | $-SCH_3$ | 136° C |
| 29 | 3-Cl | 5-Cl | $3-CF_3$ | H | H | $-OC_2H_5$ | 132° C |
| 30 | 3-Cl | H | 3-Cl | $-CH_3$ | H | $-SCH_3$ | 119° C |
| 31 | $2-CH_3$ | H | 4-Cl | H | H | $-SCH_2COOC_2H_5$ | $n_D^{30}$ 1,6421 |
| 32 | $4-CH_3$ | H | 4-Cl | H | H | $-SCH_3$ | 88-90°C |
| 33 | 4-Cl | H | 4-Cl | H | H | $-SCH_3$ | 119-120°C |
| 34 | $2-CH_3$ | 4-Cl | 4-Cl | H | H | $-SCH_3$ | 107-110°C |
| 35 | $2-CH_3$ | $4-CH_3$ | 4-Cl | H | H | $-SCH_3$ | 67-69°C |
| 36 | $4-C(CH_3)_3$ | H | $2-CH_3$ | H | H | $-SCH_3$ | $n_D^{20}$ 1,6409 |
| 37 | 2-Cl | 4-Cl | $2-CH_3$ | H | H | $-SCH_3$ | $n_D^{20}$ 1,6614 |
| 38 | 2-Cl | 5-Cl | $2-CH_3$ | H | H | $-SCH_3$ | $n_D^{20}$ 1,6659 |

Tabelle 2 (Fortsetzung)

| Bsp.-Nr. | $R^1$ | $R^2$ | $R^3$ | $R^4$ | $R^5$ | Z | phys. Konstante ($^1$H-NMR* bzw. Schmelzpunkt) |
|---|---|---|---|---|---|---|---|
| 39 | 3-Cl | 4-Cl | 2-$CH_3$ | H | H | -$SCH_3$ | 99°C |
| 40 | 2-$CH_3$ | H | 4-Cl | H | -$CH_3$ | -$SCH_3$ | 88-91°C |
| 41 | 2-$CH_3$ | H | 4-Cl | H | H | Cl | 88-90° C |
| 42 | 2-$CH_3$ | H | 4-Cl | -$CH_3$ | H | -$SCH_3$ | Öl |
| 43 | 2-$CH_3$ | H | 4-Cl | H | H | -Br | 92-95° C |
| 44 | 2-$CH_3$ | H | 4-Cl | H | H | -$C_3H_7$-n | $n_D^{20}$ 1,6259 |
| 45 | 2-$CH_3$ | H | 4-Cl | H | H | -$OCH_2$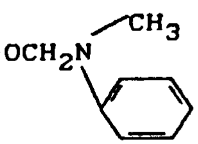 | $n_D^{30}$ 1,6259 |
| 46 | 2-$CH_3$ | H | 4-Cl | H | H | -C≡C-Si$(CH_3)_3$ | 68-70°C |
| 47 | 2-$CH_3$ | H | 4-Cl | H | H | -C≡CH | 75-77°C |
| 48 | 2-$CH_3$ | 4-$CH_3$ | 3-Cl | H | H | -$SC_2H_5$ | 2.45 (s, 3H) |
| 49 | 2-$CH_3$ | 4-$CH_3$ | 3-$CF_3$ | H | H | -$SC_2H_5$ | 2.4 (s, 3H) |
| 50 | 2-F | 4-F | 3-Cl | H | H | -$OCH_3$ | 101° C |
| 51 | 2-$CH_3$ | H | 3-Cl | $CH_3$ | H | -$SCH_3$ | 2.4 (s, 3H) |
| 52 | 2-F | 4-F | 3-Cl | H | H | -$SC_2H_5$ | 65° C |

Tabelle 2 (Fortsetzung)

| Bsp.-Nr. | $R^1$ | $R^2$ | $R^3$ | $R^4$ | $R^5$ | Z | phys. Konstante ($^1$H-NMR* bzw. Schmelzpunkt) |
|---|---|---|---|---|---|---|---|
| 53 | 2-Cl | 4-Cl | $3-CF_3$ | H | H | $-SC_2H_5$ | 76° C |
| 54 | 2-Cl | 4-Cl | 3-Cl | H | H | $-SC_2H_5$ | 86° C |
| 55 | 2-F | 4-F | 3-F | H | H | $-SCH_3$ | 2,6 (s, 3H) |
| 56 | 2-Cl | 4-Cl | $3-CF_3$ | H | H | $-OCH_3$ | 105° C |
| 57 | 2-Cl | 4-Cl | $3-CF_3$ | H | H | $-OC_3H_7n$ | 94° C |
| 58 | 2-F | 4-F | $3-CF_3$ | H | H | $-OCH_2-C{\equiv}CH$ | 4,9 (d, 2H) |
| 59 | 2-Cl | 4-Cl | $3-CF_3$ | H | H | $-OC_2H_5$ | 87° C |
| 60 | 3-Cl | H | 4-CN | H | H | $-SCH_3$ | 152° C |
| 61 | 2-Cl | H | 4-CN | H | H | $-SCH_3$ | 137° C |
| 62 | 4-Cl | H | 3-Cl | $CH_3$ | H | $-OC_2H_5$ | 2,2(s, 3H) |
| 63 | 2-F | 4-F | $3-CF_3$ | H | H | $-SC_2H_5$ | 57° C |
| 64 | $2-CH_3$ | $4-CH_3$ | $3-CF_3$ | H | H | $-SCH_3$ | 2,6 (s, 3H) |
| 65 | 2-F | 4-F | $3-CF_3$ | H | H | $-OCH_2-CH=CH_2$ | 4,7 (m, 2H) |
| 66 | 2-Cl | 4-Cl | $3-CF_3$ | H | H | $-SCH_3$ | 117° C |
| 67 | 4-Cl | H | 3-Cl | $CH_3$ | H | $-SCH_3$ | 98° C |
| 68 | 2-F | 4-F | 3-Cl | H | H | $-OC_2H_5$ | 68° C |
| 69 | 2-Cl | 4-Cl | 3-Cl | H | H | $-OC_2H_5$ | 119° C |

Tabelle 2 (Fortsetzung)

| Bsp.-Nr. | $R^1$ | $R^2$ | $R^3$ | $R^4$ | $R^5$ | Z | phys. Konstante ($^1$H-NMR* bzw. Schmelzpunkt) |
|---|---|---|---|---|---|---|---|
| 70 | $3-CF_3$ | $5-CF_3$ | 2-Cl | H | H | $-SCH_3$ | 135° C |
| 71 | 3-Cl | 5-Cl | 3-Cl | H | H | $-OC_2H_5$ | 140° C |
| 72 | 3-Cl | H | 3-Cl | $CH_3$ | H | $-OC_2H_5$ | 2.2 (s, 3H) |
| 73 | $2-CH_3$ | $4-CH_3$ | $3-CF_3$ | H | H | $-OCH_3$ | 3.95 (s, 3H) |
| 74 | $2-CH_3$ | $4-CH_3$ | $3-CF_3$ | H | H | $-OC_2H_5$ | 2.45 (s, 3H) |
| 75 | 2-F | 4-F | 3-F | H | H | $-OCH_3$ | 84° C |
| 76 | $2-CH_3$ | $4-CH_3$ | $3-CF_3$ | H | H | $-OC_3H_7n$ | 2.45 (s, 3H) |
| 77 | 2-F | 4-F | 3-F | H | H | $-OC_2H_5$ | 99° C |
| 78 | 2-Cl | 4-Cl | $3-CF_3$ | H | H | 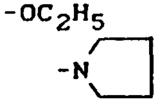 | 152° C |
| 79 | $2-CH_3$ | $4-CH_3$ | 3-Cl | H | H | $-SCH_3$ | 72° C |

* Die $^1$H-NMR Spektrum wurden in Deuterochloroform ($CDCl_3$) mit Tetramethylsilan (TMS) als innerem Standard aufgenommen. Angegeben ist die chemische Verschiebung als δ-Wert in ppm.

<u>Tabelle 2</u> (Fortsetzung)

| Bsp.-Nr. | $R^1$ | $R^2$ | $R^3$ | $R^4$ | $R^5$ | Z | phys. Konstante ($^1$H-NMR* bzw. Schmelzpunkt) |
|---|---|---|---|---|---|---|---|
| 80 | 2-$CH_3$ | 4-$CH_3$ | 3-$CF_3$ | H | H | 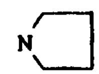 | 98° C |
| 81 | 2-Cl | 4-Cl | 3-Cl | H | H | $OCH_3$ | 132° C |
| 82 | 2-F | 4-F | 4-F | H | H | $SCH_3$ | 78° C |
| 83 | 2-F | 4-F | 2-Cl | H | H | $OCH_3$ | 92° C |
| 84 | 2-$CH_3$ | 4-$CH_3$ | 2-Cl | H | H | $OCH_3$ | 3.95 (s, 3H) |
| 85 | 2-F | 4-F | 2-Cl | H | H | $OC_2H_5$ | 79° C |
| 86 | 2-$CH_3$ | 4-$CH_3$ | 3-$CF_3$ | H | H | $NHC_3H_7n$ | 1.7 (m, 2H) |
| 87 | 2-$CF_3$ | 4-$CH_3$ | 3-Cl | H | H | $OC_2H_5$ | 4.2 (q, 2H, J=7Hz) |
| 88 | 2-F | 4-F | 2-$CH_3$ | H | H | $SCH_3$ | 2.6 (s, 3H) |
| 89 | 2-$CH_3$ | 4-$CH_3$ | 3-Cl | H | H | 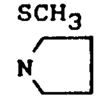 | 3.4 (m, 4H) |
| 90 | 2-Cl | 4-Cl | 3-Cl | H | H | 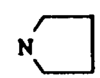 | 135° C |

**Tabelle 2** (Fortsetzung)

| Bsp.-Nr. | $R^1$ | $R^2$ | $R^3$ | $R^4$ | $R^5$ | Z | phys. Konstante ($^1$H-NMR* bzw. Schmelzpunkt) |
|---|---|---|---|---|---|---|---|
| 91 | 2-F | 4-F | 4-F | H | H | $OC_2H_5$ | 109° C |
| 92 | 2-Cl | H | 3-CN | H | H | $OCH_3$ | 141° C |
| 93 | 4-Cl | H | 3-CN | H | H | $OCH_3$ | 158° C |
| 94 | 2-F | 4-F | 2-$CH_3$ | H | H | $OC_2H_5$ | 4.2 (q, 2H, J=7Hz) |
| 95 | 2-F | 4-F | 2-$CH_3$ | H | H | $OCH_3$ | 3.95 (s, 3H) |
| 96 | 3-Cl | H | 4-CN | H | H | $OC_2H_5$ | 4.25 (q, 2H, J=7HZ) |
| 97 | 2-Cl | H | 4-CN | H | H | $OC_2H_5$ | 138° C (Zers.) |
| 98 | 2-Cl | H | 3-CN | H | H | $SCH_3$ | 130° C |
| 99 | 4-Cl | H | 3-CN | H | H | $SCH_3$ | 139° C |
| 100 | 2-Cl | H | 3-$OC_2H_5$ | H | H | $OC_2H_5$ | 160° C |
| 101 | 4-Cl | H | 3-CN | H | H | $OC_2H_5$ | 217°C |
| 102 | 4-$CF_3$ | H | 3-CN | H | H | $SCH_3$ | 181°C |
| 103 | 4-$CF_3$ | H | 3-CN | H | H | $OC_2H_5$ | 186°C |
| 104 | 4-$CF_3$ | H | 3-CN | H | H | $OCH_3$ | 186°C |

Herstellung der Ausgangsverbindungen

Beispiel (III-1)

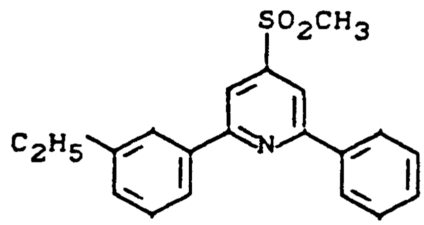

2,7 g (0,009 mol) 4-Methylthio-2-(3-ethyl-phenyl)-6-phenyl-pyridin werden in 30 ml trockenem Methylenchlorid mit 0,8 g (0,018 mol) Ameisensäure und 0,1 g Ammoniummolybdat als Katalysator versetzt und unter heftigem Rühren werden 2,6 g (0,027 mol) 35 %ige Wasserstoffperoxid-Lösung bei Raumtemperatur zugetropft. Die Mischung wird über Nacht bei Raumtemperatur gerührt, getrennt, die organische Phase mit Natriumhydrogensulfit-Lösung gewaschen, mit Natriumsulfat getrocknet und eingeengt. Der Rückstand wird mit Ether verrührt, abgesaugt und getrocknet.

Man erhält 1,7 g (57 % der Theorie) 4-Methylsulfonyl-2-(3-ethyl-phenyl)-6-phenyl-pyridin vom Schmelzpunkt 131 °C.

Analog zu dem Herstellungsbeispiel (III-1) und gemäß den allgemeinen Angaben zu den erfindungsgemäßen Verfahren erhält man die in Tabelle 3 aufgeführten Verbindungen der allgemeinen Formel (III)

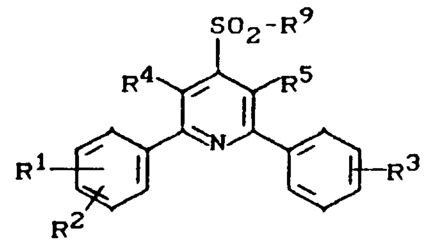

(III)

Tabelle 3

| Bsp.-Nr. | $R^1$ | $R^2$ | $R^3$ | $R^4$ | $R^5$ | $R^9$ | Schmelzpunkt |
|---|---|---|---|---|---|---|---|
| (III-2) | $3-CF_3$ | H | $2-OCH_3$ | H | H | $CH_3$ | 115° C |
| (III-3) | $3-CF_3$ | H | $3-C_2H_5$ | H | H | $CH_3$ | |
| (III-4) | $3-CF_3$ | H | $4-C_2H_5$ | H | H | $CH_3$ | 142° C |
| (III-5) | 3-Cl | 5-Cl | H | H | H | $CH_3$ | |
| (III-6) | 2-F | 4-F | $3-CF_3$ | H | H | $CH_3$ | |
| (III-7) | 3-Cl | 5-Cl | H | H | H | $CH_3$ | |
| (III-8) | 3-Cl | 5-Cl | $2-OCH_3$ | H | H | $CH_3$ | |
| (III-9) | 3-Cl | 5-Cl | $3-CF_3$ | H | H | $CH_3$ | |
| (III-10) | 3-Cl | H | H | $CH_3$ | H | $CH_3$ | |
| (III-11) | 2-F | 4-F | 3-Cl | H | H | $CH_3$ | |
| (III-12) | 2-Cl | 4-Cl | 3-Cl | H | H | $CH_3$ | |
| (III-13) | 3-Cl | H | 3-Cl | $CH_3$ | H | $CH_3$ | |

Tabelle 3 (Fortsetzung)

| Bsp.-Nr. | $R^1$ | $R^2$ | $R^3$ | $R^4$ | $R^5$ | $R^9$ | Schmelzpunkt |
|---|---|---|---|---|---|---|---|
| (III-14) | 3-Cl | 5-Cl | 3-Cl | H | H | $CH_3$ | |
| (III-15) | 2-Cl | 4-Cl | 3-$CF_3$ | H | H | $CH_3$ | |
| (III-16) | 2-$CH_3$ | 4-$CH_3$ | 3-Cl | H | H | $CH_3$ | 130° C |
| (III-17) | 3-$CF_3$ | 5-$CF_3$ | 2-$CH_3$ | H | H | $CH_3$ | |
| (III-18) | 3-$CF_3$ | 5-$CF_3$ | 2-Cl | H | H | $CH_3$ | |
| (III-19) | 3-Cl | H | 4-Cl | H | $CH_3$ | $CH_3$ | |
| (III-20) | 2-$CH_3$ | 4-$CH_3$ | 3-$CF_3$ | H | H | $CH_3$ | |
| (III-21) | 2-F | 4-F | 3-F | H | H | $CH_3$ | |
| (III-22) | 2-F | 4-F | 2-Cl | H | H | $CH_3$ | 85° C |
| (III-23) | 2-F | 4-F | 4-F | H | H | $CH_3$ | 198° C |
| (III-24) | 3-Cl | H | 4-CN | H | H | $CH_3$ | |
| (III-25) | 2-Cl | H | 4-CN | H | H | $CH_3$ | |
| (III-26) | 4-Cl | H | 3-CN | H | H | $CH_3$ | 174° C |
| (III-27) | 2-Cl | H | 3-CN | H | H | $CH_3$ | 140° C |

Beispiel XI-1:

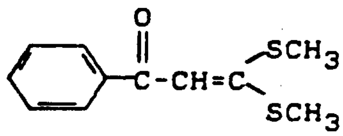

Zu 30 g (0,25 mol)Acetophenon in 300 ml trockenem Dimethylformamid werden 20,1 g (0,265 mol) Schwefelkohlenstoff und dann portionsweise 16,5 g (0,55 mol) einer 80 %igen Natriumhydrid-Öl-Suspension gegeben, wobei die Temperatur auf ca. 40 °C ansteigt. Die Mischung wird weitere 30 Minuten bei Raumtemperatur gerührt, dann werden 88,8 g (0,625 mol) Methyliodid während 30 Minuten zugetropft und die Mischung weitere 2 bis 3 Stunden bei Raumtemperatur gerührt. Nach der Zugabe von 600 ml Wasser fällt ein Niederschlag aus, der abgesaugt, mit Wasser gewaschen und getrocknet wird. Nach dem Umkristallisieren aus i-Propanol erhält man 31,4 g (56 % der Theorie) 3,3-Bis-(methylthio)-1-phenyl-2-propen-1-on vom Schmelzpunkt 92 °C.

Auf analoge Weise können die in der nachfolgenden Tabelle 4 aufgeführten Verbindungen der Formel (XI) erhalten werden:

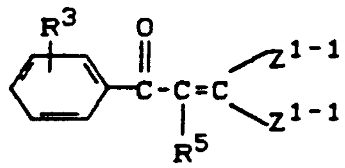

(XI)

__Tabelle 4__

| Bsp.-Nr. | $R^3$ | $R^5$ | $Z^{1-1}$ | physikalische Konstante |
|---|---|---|---|---|
| XI-2 | 2-$CH_3$ | H | $SCH_3$ | |
| XI-3 | 4-Cl | H | $SCH_3$ | |
| XI-4 | 2-$OCH_3$ | H | $SCH_3$ | |
| XI-5 | 4-Cl | $CH_3$ | $SCH_3$ | |
| XI-6 | 2-Cl | H | $SCH_3$ | |
| XI-7 | 2-$CF_3$ | H | $SCH_3$ | |

__Anwendungsbeispiele__

__Beispiel A__

Post-emergence-Test (Gewächshaus)

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine sehr gute Wirksamkeit und Nutzpflanzenselektivität zeigen in diesem Test z.B. die Verbindungen gemäß folgender Herstellungsbeispiele 2, 3, 10, 15 und 19.

## Patentansprüche

**1.** 2,6-Diarylpyridin-Derivate der allgemeinen Formel (I),

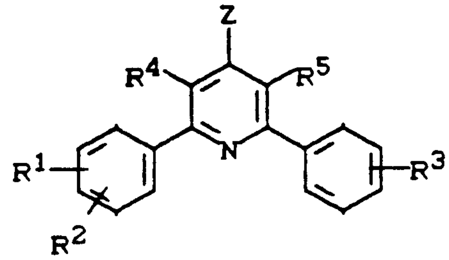

(I)

in welcher

$R^1$ und $R^2$ jeweils unabhängig voneinander für Wasserstoff, Halogen, Alkyl, Alkoxy oder Halogenalkyl stehen,

$R^3$ für Wasserstoff, Halogen, Cyano, Alkyl, Alkoxy oder Halogenalkyl steht,

$R^4$ und $R^5$ jeweils unabhängig voneinander für Wasserstoff oder Alkyl stehen und

Z für Alkyl, Alkenyl, Alkinyl, Alkoxy, Alkenyloxy, Alkinyloxy, Alkylthio, Alkenylthio, Alkinylthio, Alkoxycarbonylalkylthio, Trimethylsilylalkinyl, Halogen oder für eine der folgenden Gruppierungen

$-NH-R^6$;

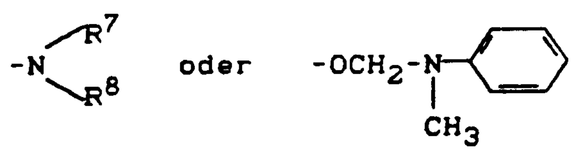

steht, wobei

$R^6$ und $R^7$ für Alkyl, Alkenyl oder Alkinyl stehen,

$R^8$ für Alkyl steht oder

$R^7$ und $R^8$ gemeinsam mit dem Stickstoffatom, an welches sie gebunden sind, für eine gegebenenfalls durch Sauerstoff, Schwefel oder für durch Alkyl substituierten Stickstoff unterbrochene gesättigte Alkylkette stehen,

wobei die Verbindungen ausgenommen sind, in denen

$R^4$ und $R^5$ gleichzeitig für Wasserstoff stehen,

Z für Alkoxy oder Alkylthio steht und

a) $R^1$ und $R^2$ gleichzeitig für Wasserstoff stehen oder

b) einer der Reste $R^1$ oder $R^2$ für Wasserstoff steht und der andere Rest $R^2$ bzw. $R^1$ in der ortho- oder meta-Position des Phenyl für Halogen, Methyl oder Trifluormethyl steht,

und wobei die Verbindungen

2,6-Bis(4-bromphenyl)-4-methylthio-pyridin,

2,6-Bis(4-methoxyphenyl)-4-methylthio-pyridin,

4-n-Butyl-2,6-diphenyl-pyridin,
4-(1-Methylethyl)-2,6-diphenyl-pyridin,
2,6-Bis(4-methoxyphenyl)-4-methyl-pyridin,
4-Methyl-2,6-diphenyl-pyridin,
2,6-Bis(p-chlorphenyl)-4-picolin und
4-Ethyl-2,6-diphenyl-pyridin
ausgenommen sind.

**2.** 2,6-Diarylpyridin-Derivate der Formel (I) gemäß Anspruch 1,

$R^1$ für Wasserstoff, Halogen, Alkyl oder Alkoxy mit jeweils 1 bis 6 Kohlenstoffatomen oder für Halogenalkyl mit 1 bis 6 Kohlenstoffatomen und 1 bis 13 gleichen oder verschiedenen Halogenatomen steht,

$R^2$ für Wasserstoff, Halogen, Alkyl oder Alkoxy mit jeweils 1 bis 6 Kohlenstoffatomen oder für Halogenalkyl mit 1 bis 6 Kohlenstoffatomen und 1 bis 13 gleichen oder verschiedenen Halogenatomen steht,

$R^3$ für Wasserstoff, Halogen, Cyano, Alkyl oder Alkoxy mit jeweils 1 bis 6 Kohlenstoffatomen oder für Halogenalkyl mit 1 bis 6 Kohlenstoffatomen und 1 bis 13 gleichen oder verschiedenen Halogenatomen steht,

$R^4$ für Wasserstoff oder Alkyl mit 1 bis 6 Kohlenstoffatomen steht,

$R^5$ für Wasserstoff oder Alkyl mit 1 bis 6 Kohlenstoffatomen steht,

Z für Alkyl mit 1 bis 8 Kohlenstoffatomen, Alkoxy oder Alkylthio mit jeweils 1 bis 8 Kohlenstoffatomen, oder für Alkenyl, Alkenyloxy oder Alkenylthio mit jeweils 2 bis 6 Kohlenstoffatomen, Alkinyl, Alkinyloxy oder Alkinylthio mit jeweils 2 bis 6 Kohlenstoffatomen, Alkoxycarbonylalkylthio mit jeweils 1 bis 4 Kohlenstoffatomen im Alkoxy-oder Alkylteil, Trimethylsilylalkinyl mit 2 bis 4 Kohlenstoffatomen im Alkinylteil, Halogen oder für eine der folgenden Gruppierungen
$-NH-R^6$;

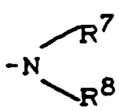
oder
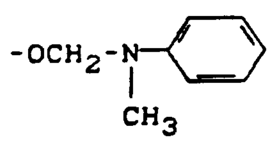

steht, wobei

$R^6$ und $R^7$ unabhängig voneinander für Alkyl mit 1 bis 6 Kohlenstoffatomen, Alkenyl mit 2 bis 6 Kohlenstoffatomen oder Alkinyl mit 2 bis 6 Kohlenstoffatomen steht,

$R^8$ für Alkyl mit 1 bis 6 Kohlenstoffatomen steht oder

$R^7$ und $R^8$ gemeinsam mit dem Stickstoffatom, an welches sie gebunden sind, einen 5- oder 6-gliedrigen gesättigten Ring bilden, welcher gegebenenfalls ein Sauerstoff-, Schwefel- oder Stickstoffatom enthält,
wobei das Stickstoffatom durch $C_1$-$C_4$-Alkyl substituiert sein kann,

ausgenommen die in Anspruch 1 genannten Verbindungen.

**3.** 2,6-Diarylpyridin-Derivate der Formel (I) gemäß Anspruch 1, in welcher

$R^1$ für Wasserstoff, Fluor, Chlor, Brom, Methyl, Ethyl,n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy oder für Halogenalkyl mit 1 bis 4 Kohlenstoffatomen und 1 bis 9 Fluor- und/oder Chloratomen steht,

$R^2$ für Wasserstoff, Fluor, Chlor, Brom, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy oder für Halogenalkyl mit 1 bis 4 Kohlenstoffatomen und 1 bis 9 Fluor- und/oder Chloratomen steht,

$R^3$ für Wasserstoff, Fluor, Chlor, Brom, Cyano, Methyl, Ethyl, n-Propyl, i-Propyl, n-Butyl-, i-Butyl, s-Butyl, t-Butyl, Methoxy, Ethoxy, n-Propoxy, i-Propoxy, n-Butoxy, i-Butoxy, s-Butoxy, t-Butoxy oder für Halogenalkyl mit 1 bis 4 Kohlenstoffatomen und 1 bis 9 Fluor- und/oder Chloratomen steht,

$R^4$ und $R^5$ unabhängig voneinander für Wasserstoff, Methyl, Ethyl, n-Propyl, i-Propyl, n-Butyl, i-Butyl, s-Butyl oder t-Butyl stehen;

Z für Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy, Methylthio, Ethylthio, n-Propylthio, i-Propylthio, n-Butylthio, s-Butylthio, i-Butylthio, t-Butylthio, für Alkenyl, Alkenyloxy oder Alkenylthio mit jeweils 2 bis 4 Kohlenstoffatomen, Alkinyl, Alkinyloxy oder Alkinylthio mit 2 bis 4 Kohlenstoffatomen, Alkoxycarbonylalkylthio mit 1 bis 4 Kohlenstoffatomen im Alkoxy- und 1 oder 2 Kohlenstoffatomen im Alkylteil, Trimethylsilylalkinyl mit 2 bis 4 Kohlenstoffatomen im Alkinylteil, Fluor, Chlor, Brom oder für eine der folgenden Gruppierungen

-NH-$R^6$;

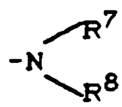 oder 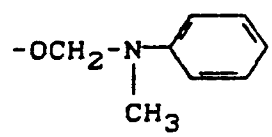

steht, wobei

$R^6$ und $R^7$ unabhängig voneinander für Methyl, Ethyl, n-Propyl, i-Propyl, n-Butyl, i-Butyl, s-Butyl, t-Butyl, Alkenyl mit 2 bis 4 Kohlenstoffatomen oder Alkinyl mit 2 bis 4 Kohlenstoffatomen steht,

$R^8$ für Methyl, Ethyl, n-Propyl, i-Propyl, n-Butyl, i-Butyl, s-Butyl oder t-Butyl steht oder

$R^7$ und $R^8$ gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält, wobei das Stickstoffatom durch Methyl, Ethyl, n-oder i-Propyl substituiert sein kann,

ausgenommen die in Anspruch 1 oben genannten Verbindungen.

**4.** Verfahren zur Herstellung von 2,6-Diarylpyridin-Derivaten der Formel (I),

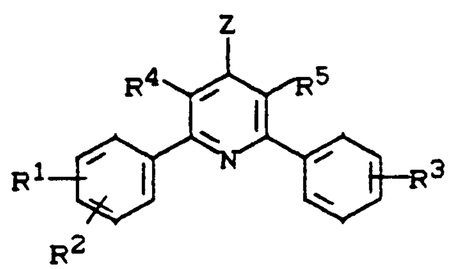

(I)

in welcher

$R^1$ und $R^2$ jeweils unabhängig voneinander für Wasserstoff, Halogen, Alkyl, Alkoxy oder Halogenalkyl stehen,

$R^3$ für Wasserstoff, Halogen, Cyano, Alkyl, Alkoxy oder Halogenalkyl steht,

$R^4$ und $R^5$ jeweils unabhängig voneinander für Wasserstoff oder Alkyl stehen und

Z für Alkyl, Alkenyl, Alkinyl, Alkoxy, Alkenyloxy, Alkinyloxy, Alkylthio, Alkenylthio, Alkinylthio, Alkoxycarbonylalkylthio, Trimethylsilylalkinyl, Halogen oder für eine der folgenden Gruppierungen

-NH-$R^6$;

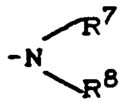

oder

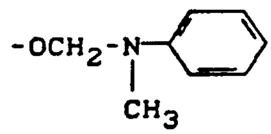

steht, wobei

$R^6$ und $R^7$ für Alkyl, Alkenyl oder Alkinyl stehen,

$R^8$ für Alkyl steht oder

$R^7$ und $R^8$ gemeinsam mit dem Stickstoffatom, an welches sie gebunden sind, für eine gegebenenfalls durch Sauerstoff, Schwefel oder für durch Alkyl substituierten Stickstoff unterbrochene gesättigte Alkylkette stehen,

wobei die Verbindungen ausgenommen sind, in denen

$R^4$ und $R^5$ gleichzeitig für Wasserstoff stehen,

Z für Alkoxy oder Alkylthio steht und

a) $R^1$ und $R^2$ gleichzeitig für Wasserstoff stehen oder

b) einer der Reste $R^1$ oder $R^2$ für Wasserstoff steht und der andere Rest $R^2$ bzw. $R^1$ in der ortho- oder meta-Position des Phenyl für Halogen, Methyl oder Trifluormethyl steht,

und wobei die Verbindungen
2,6-Bis(4-bromphenyl)-4-methylthio-pyridin,
2,6-Bis(4-methoxyphenyl)-4-methylthio-pyridin,
4-n-Butyl-2,6-diphenyl-pyridin,
4-(1-Methylethyl)-2,6-diphenyl-pyridin,
2,6-Bis(4-methoxyphenyl)-4-methyl-pyridin,
4-Methyl-2,6-diphenyl-pyridin,
2,6-Bis(p-chlorphenyl)-4-picolin und
4-Ethyl-2,6-diphenyl-pyridin
ausgenommen sind,

dadurch gekennzeichnet, daß man

a) die erfindungsgemäßen 2,6-Diarylpyridin-Derivate der Formel (Ia) erhält,

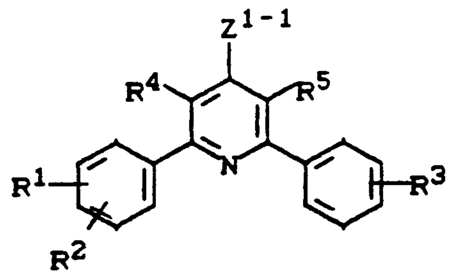

(Ia)

in welcher

$R^1$, $R^2$, $R^3$, $R^4$ und $R^5$ die oben angegebene Bedeutung haben und

$Z^{1-1}$ für Alkylthio, Alkenylthio, Alkinylthio oder Alkoxycarbonylalkylthio steht,

wenn man 1,5-Dioxo-Verbindungen der Formel (II) bzw. deren Isomere der Formel (IIa)

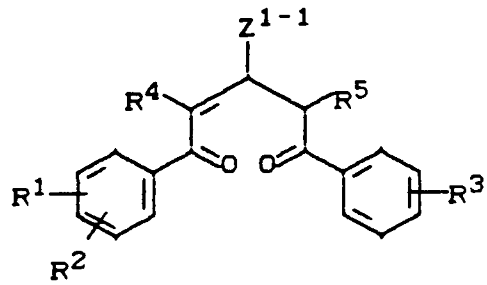

(II)

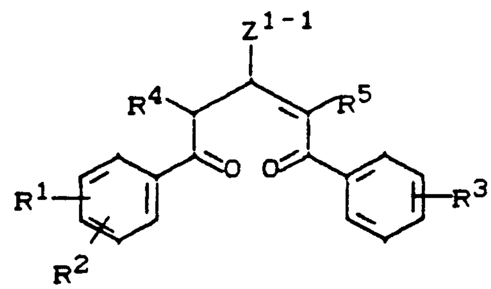

(IIa)

in welcher

$R^1$, $R^2$, $R^3$, $R^4$, $R^5$ und $Z^{1-1}$ die oben angegebene Bedeutung haben,

mit Ammoniak oder einem Ammoniumsalz gegebenenfalls in Gegenwart eines Verdünnungsmittels umsetzt, oder daß man

b) die erfindungsgemäßen 2,6-Diarylpyridin-Derivate der Formel (Ib) erhält,

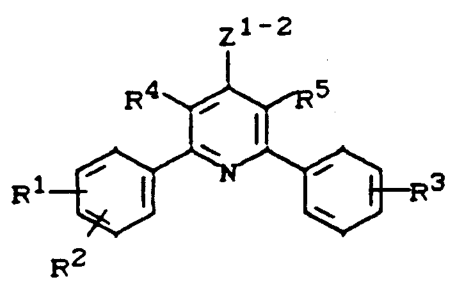

(Ib)

in welcher

$Z^{1-2}$ für Alkoxy, Alkenyloxy, Alkinyloxy oder für eine der folgenden Gruppierungen

$-NH-R^6$;

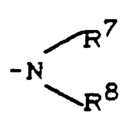

oder

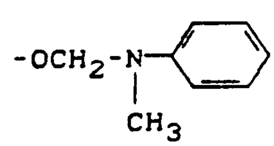

steht und

$R^1$, $R^2$, $R^3$, $R^4$ und $R^5$ die oben angegebene Bedeutung haben,

wenn man 4-Sulfonylalkyl-2,6-diarylpyridin-Derivate der Formel (III)

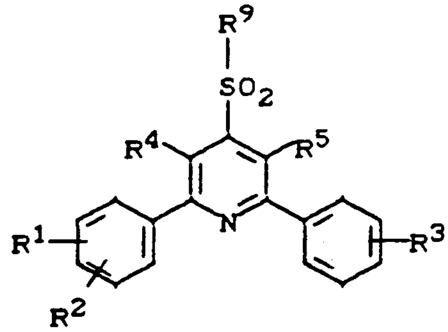

(III)

in welcher

$R^1$, $R^2$, $R^3$, $R^4$ und $R^5$ die oben angegebene Bedeutung haben und

$R^9$ für Alkyl steht, entweder

b-α) mit Verbindungen der Formel (IV)

$Z^{1-3}$-M (IV)

in welcher

$Z^{1-3}$ für Alkoxy, Alkenyloxy, Alkinyloxy oder für die Gruppe

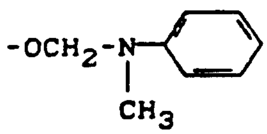

steht und

M für ein Alkalimetallkation steht,

gegebenenfalls in Gegenwart eines Verdünnungsmittels umsetzt, oder

b-β) mit Aminen der Formel (V) oder (Va)

$H_2N-R^6$ (V)

oder

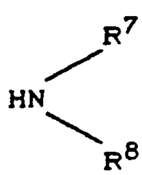

(Va)

in welcher

$R^6$, $R^7$ und $R^8$ die oben angegebene Bedeutung haben,

gegebenenfalls in Gegenwart eines Verdünnungsmittels und gegebenenfalls unter erhöhtem Druck umsetzt, oder daß man

c) 2,6-Diarylpyridin-Derivate der Formel (Ic) erhält,

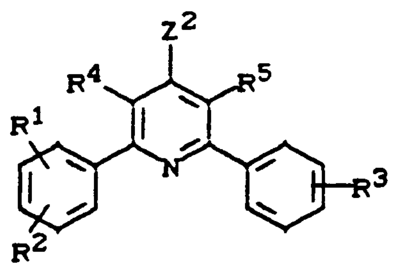

(Ic)

in welcher

$R^1$, $R^2$, $R^3$, $R^4$ und $R^5$ die oben angegebene Bedeutung haben und

$Z^2$ für Halogen steht,

wenn man 4-Hydroxy-2,6-Diarylpyridin-Derivate der Formel (VI)

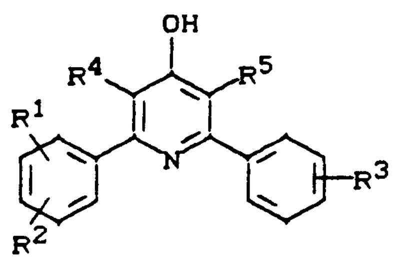

(VI)

in welcher

$R^1$, $R^2$, $R^3$, $R^4$ und $R^5$ die oben angegebene Bedeutung haben,

mit einem Halogenierungsreagenz gegebenenfalls in Gegenwart eines Verdünnungsmittels umsetzt, oder daß man

d) 2,6-Diarylpyridin-Derivate der Formel (Id) erhält,

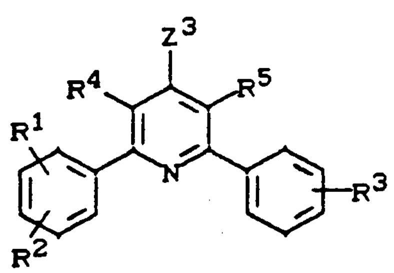

(Id)

in welcher

$R^1$, $R^2$, $R^3$, $R^4$ und $R^5$ die oben angegebene Bedeutung haben, und

$Z^3$ für Alkyl, Alkenyl, Alkinyl oder Trimethylsilylalkinyl steht,

wenn man 2,6-Diarylpyridin-Derivate der Formel (Ic)

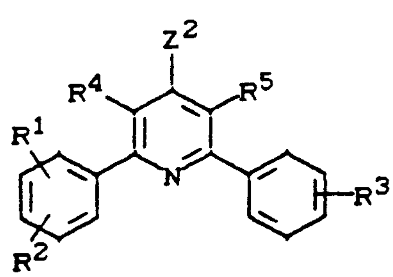

(Ic)

in welcher

$R^1$, $R^2$, $R^3$, $R^4$ und $R^5$ die oben angegebene bedeutung haben, und
$Z^2$ für Halogen steht,

mit Diester-Derivaten der Formel (VII)

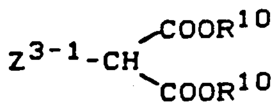

(VII)

in welcher

$Z^{3-1}$ für Wasserstoff, Alkyl, Alkenyl, Alkinyl oder Trimethylsilylalkinyl steht, und
$R^{10}$ für Methyl oder Ethyl steht,

gegebenenfalls in Gegenwart eines Verdünnungsmittels und gegebenenfalls in Gegenwart einer Base umsetzt und die so erhaltenen Verbindungen gegebenenfalls in Gegenwart eines Verdünnungsmittels und in Gegenwart einer Base hydrolysiert und anschließend in Gegenwart einer Säure decarboxyliert.

**5.** Herbizide Mittel, gekennzeichnet durch einen Gehalt an mindestens einem 2,6-Diarylpyridin-Derivat der Formel (I) gemäß den Ansprüchen 1 oder 4.

**6.** Verfahren zur Bekämpfung von unerwünschten Pflanzen, dadurch gekennzeichnet, daß man 2,6-Diarylpyridin-Derivate der Formel (I) gemäß den Ansprüchen 1 oder 4 auf die unerwünschten Pflanzen und/oder ihren Lebensraum einwirken läßt.

**7.** Verwendung von 2,6-Diarylpyridin-Derivaten der Formel (I) gemäß den Ansprüchen 1 oder 4 zur Bekämpfung von unerwünschten Pflanzen.

**8.** Verfahren zur Herstellung von herbiziden Mitteln, dadurch gekennzeichnet, daß man 2,6-Diarylpyridin-Derivate der Formel (I) gemäß den Ansprüchen 1 oder 4 mit Streckmitteln und/oder oberflächenaktiven Substanzen vermischt.

**9.** 4-Sulfonylalkyl-2,6-diarylpyridin-Derivate der Formel (III)

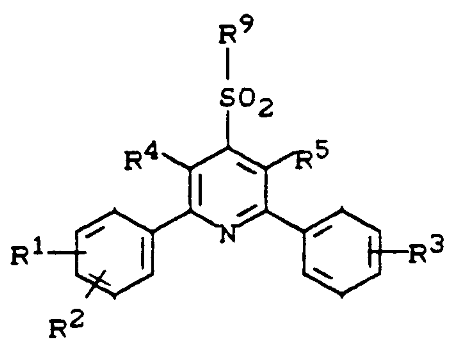

(III)

in welcher

$R^1$ und $R^2$ jeweils unabhängig voneinander für Wasserstoff, Halogen, Alkyl, Alkoxy oder Halogenalkyl stehen,
$R^3$ für Wasserstoff, Halogen, Cyano, Alkyl, Alkoxy oder Halogenalkyl steht,
$R^4$ und $R^5$ jeweils unabhängig voneinander für Wasserstoff oder Alkyl stehen und
$R^9$ für Alkyl steht.

**10.** 4-Hydroxy-2,6-diarylpyridin-Derivate der Formel (VI)

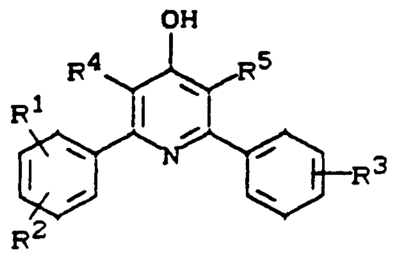

(VI)

in welcher

$R^1$ und $R^2$ jeweils unabhängig voneinander für Wasserstoff, Halogen, Alkyl, Alkoxy oder Halogenalkyl stehen,

$R^3$ für Wasserstoff, Halogen, Cyano, Alkyl, Alkoxy oder Halogenalkyl steht,

$R^4$ und $R^5$ jeweils unabhängig voneinander für Wasserstoff oder Alkyl stehen.

Europäisches Patentamt

# EUROPÄISCHER RECHERCHENBERICHT

Nummer der Anmeldung

**EP 91 10 9714**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 263 958 (SUMITOMO CHEMICAL CO. LTD)<br>* whole document *<br>– – – | 1-3 | C 07 D 213/68<br>C 07 D 213/71<br>C 07 D 213/74 |
| A | US-A-4 451 659 (POTTS et al.)<br>* whole document *<br>– – – – – | 4 | |
| | | | RECHERCHIERTE SACHGEBIETE (Int. Cl.5) |
| | | | C 07 D 213/00 |

Der vorliegende Recherchenbericht wurde für alle Patentansprüche erstellt

| Recherchenort | Abschlußdatum der Recherche | Prüfer |
|---|---|---|
| Berlin | 23 September 91 | FRELON D.L.M.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument